# EUROPEAN PATENT SPECIFICATION

(11) **EP 4 234 609 B1**
(45) Date of publication and mention of the grant of the patent: **17.07.2024**
(21) Application number: 22305218.4
(22) Date of filing: 25.02.2022
(51) Int. Cl.: C08G 73/02, C12Q 1/70, A61K 47/59, C12N 15/861, G01N 1/34

(54) **METHODS FOR PURIFICATION, DETECTION AND QUANTIFICATION OF RESIDUAL PEI-BASED TRANSFECTION REAGENTS**
VERFAHREN ZUR REINIGUNG, DETEKTION UND QUANTIFIZIERUNG VON RÜCKSTÄNDEN VON PEI-BASIERTEN TRANSFEKTIONSREAGENZIEN
PROCÉDÉS DE PURIFICATION, DE DÉTECTION ET DE QUANTIFICATION DE RÉACTIFS DE TRANSFECTION RÉSIDUELS À BASE DE PEI

(43) Date of publication of application: 30.08.2023
(73) Proprietor: PolyPlus Transfection, 67400 Illkirch-Graffenstaden (FR)
(72) Inventor: HELLAL, Malik, 67400 ILLKIRCH-GRAFFENSTADEN (FR); ERBACHER, Patrick, 67230 BENFELD (FR); PHILIPSON, Yann, 67800 BISCHHEIM (FR); FRANCK, Sébastien, 67118 GEISPOLSHEIM (FR); WOHLFROM, Elodie, 67640 FEGERSHEIM (FR)
(74) Representative: Ernest Gutmann - Yves Plasseraud S.A.S.

(56) References cited:
- WO-A1-2021/023796
- ANONYMOUS: "Process Impurity Testing: Polyethylenimine (PEI) | Process and Product Impurities | Assay Packages | BioReliance Product Characterization Services | Merck", 20 September 2019 (2019-09-20), XP055949741, Retrieved from the Internet <URL:https://www.merckmillipore.com/DE/de/20190920_173402> [retrieved on 20220808]

## Description

The present invention relates to methods for purification, detection and quantification of residual PEI-based transfection reagents. The present invention is directed to a method for performing an acidic hydrolysis of a liquid mixture comprising a biological matrix and a polyethyleneimine (PEI)-based transfection reagent of general formula (I) as described herein, wherein the biological matrix comprises a recombinant virus or virus-like particles produced using the PEI-based transfection reagent, and wherein said acidic hydrolysis does not degrade the PEI-based transfection reagent. The present invention also relates to a method for purifying, detecting and/or quantifying a PEI-based transfection reagent of general formula (I) as described herein.

Gene therapy aims to provide an exogenous gene in a cell to correct the expression of a deficient gene in monogenic diseases or to provide a new beneficial gene expression in cancer, viral and immune infections. To achieve that, an exogenous genetic material must be introduced efficiently into the cell correcting where the gene expression will trigger the expected therapeutic effect.

The exogenous genetic material consists of a gene expression system comprising the gene of interest framed by sequences controlling its expression (promoters, enhancer, exon, etc.) and presents on a nucleic acid construct (plasmid, DNA linear or ligated double-stranded, messenger RNA, etc.).

As eukaryotic cells are not permeable to nucleic acids, delivery systems or vectors allowing their introduction have been developed. Several systems based on viral or non-viral vectors are potent delivery systems. Therefore, among them, viral vectors represent the majority of delivery systems used in clinical applications.

Viral vectors have exploited the ability of viruses to target cells, in particular to infect cells, with great efficiency linked to biological evolution for millions of years. Taking advantage of their efficiency, viral vectors, in particular recombinant viral vectors have been constructed to maintain their capacity for cell targeting or infection but suppressing their pathogenicity while using them as a genomic sequence transporter. Among the recombinant viral vectors, AAVs, retroviruses, lentiviruses and adenoviruses are the most used in gene and cell therapy. This list is not exhaustive (for review, see 'Viral Vectors in Gene Therapy', Kenneth Lundstrom, Diseases 2018, 6, 42) and depends on the progress and knowledge of other viruses and their potential use as a non-pathogenic genomic expression vector.

Viral vectors, in particular recombinant viral vectors are produced in cellular systems allowing their reproduction and multiplication, while avoiding the generation of wild type virus (for reviews, see Forsberg et al., Key Considerations in Gene Therapy Manufacturing for Commercialization, 2018*).*

Several cellular systems are used such as HEK293 cells, HeLa cells, or insect cells for the production of recombinant viral vectors. The process consists in introducing in the cell the genetic material of the recombinant virus necessary for the replication of its recombinant genome, the production of viral capsids and the encapsidation of the recombinant viral genome in its capsids, then for the recovery of the viral vectors produced.

Several methods are available to introduce the different components necessary for the production of a recombinant virus such as infection by baculovirus, transfection by chemical method (calcium phosphate or transfection reagent or formulation), physical method such as electroporation, or even generation of a stable cellular clone (integration into the host genome of the producing cell of the various viral genomic parts necessary for the production of the recombinant viruses).

The transfection by chemical method represents the method mainly used to produce the viruses used in gene and cell therapy, in particular AAVs or lentiviruses. The use of the calcium phosphate precipitation method, unsuitable for large-scale productions (production volume) due to the difficulty of controlling and reproducing nucleic acid precipitation, has been supplanted by the use of transfection reagent such as cationic polymers or cationic lipids. Among the cationic polymers, the PEI polymer is widely used for the production of recombinant viral vectors from HEK293 cells and derivatives.

Transfection allows the introduction of different nucleic acids, usually in the form of plasmid(s) as gene expression vectors into the cells necessary for the production of a recombinant virus. Transfection allows the introduction of viral genes and their transient expression over a few days (1 to 7 days) in virus-producing cells.

Several methods of polyethyleneimine's detection have been developed using spectrophotometric (Francesca Ungaro, et al. J. Pharm. Biomed. Anal. 31 (2003) 143), colorimetric (Qu, Fei Li, et al. Langmuir 2013, 29, 1199-1205) or fluorescent (Yunyi Zhang, et al. Analytical and Bioanalytical Chemistry 2017, 409, 4771*)* methods. These methods, based on specific interaction between PEI and metals (Copper, silver) (Yu Ling, et al. J. Phys. Chem. C 2015, 119, 27173-27177*)* are applicable to a solution which are not containing others nitrogen atoms able to chelate metals. These tests requiring extracting PEI from the sample matrix are not adapted to a composition comprising viral vectors, as some residual transfection reagent may be trapped inside virus.

The cells producing recombinant viruses are essentially HEK293 cells, their derivatives, subclones or genetically modified or other cells such as HeLa, CHO, .... These cells can be cultured either adherent or non-adherent (in suspension) in variable culture systems, on plastic or glass supports, or in bioreactor systems.

Virus-producing cells are cultured in culture media containing serum or not, proteins, peptides, vitamins, hormones, amino acids, lipids, salts, or containing various synthetic compounds without animal origin, or containing synthetic components, such as polymers or substances allowing their proliferation and good viability and avoiding their aggregation. Culture media are well-known, and examples of compositions are described in the literature, for example in Srivastava A, et al. (J Pharm Sci. 2021 Jul;110(7):2609-2624)*.*

The first successes of gene and cell therapy have made it possible to broaden viral vectors applications from rare diseases to more common diseases. Regulatory agencies are adapting their guidelines of manufacturing to this new offeir. The methods and processes for producing viral vectors must be governed by rules to guarantee the patient safety. Regulatory agencies have put in place "guidelines" for the production of these new drugs concerning several aspects, safety, identity, quality, purity, impurity and potential of the substance (*WHO good manufacturing practices for biological products Replacement of Annex 1 of WHO Technical Report Series, No. 822*). Among these, the production process of the active substance comprising or consisting of the viral vector must be under control in terms of impurity and must be able to identify and quantify them if they can represent a safety risk.

The production of viral vectors by a transient transfection method in cells such as HEK293 cells or its derivatives consists of two phases. The introduction of nucleic acids encompassing viral and optionally additional genes (in the form of plasmids, 1 to 4 plasmids or other forms of gene expression vectors) into HEK293 cells, cultured under adherent or non-adherent conditions, in a synthetic culture medium or not, containing culture supplements, allows the production of recombinant viruses, whether or not excreted depending on the type of recombinant virus produced. This recombinant virus production step is referred to as an upstream process. It is followed by a step of harvesting the virus and then its purification until until it may be suitable for use as a drug active susbstance [Drug substance (DS) or Drug product (DP)] in a process of formulation of a drug composition , which is defined as the downstream process (J. Fraser Wright, Biotechnology Journal, 2021, 16, 2000022*;* Martinez-Molina et al., Pharmaceutics 2020, 12(11), 1051*; and* Christopher Perry and Andrea C. M. E. Rayat, Viruses 2021, 13, 268*).* At this final stage of production, additives such as acceptable pharmaceutical excipients or ingredients (*US Food and Drug Administration's (FDA's) 21 CFR 210.3(b)(8) guidance, an excipient or inactive ingredient is any component of a drug product other than the active ingredient, and EMEA Guideline on excipients in the dossier for application for marketing authorisation of medicinal product, Doc. Ref. EMEA*/*CHMP*/*QWP*/*396951*/*2006*) can be added to promote good conservation of the virus or to prevent its degradation or aggregation.

In this viral vector production process, the transfection reagent is a raw material in the upstream process which becomes a potential impurity in the final product (DS or DP) if it is not eliminated during the downstream process. The transfection reagent has hence to be identified and quantified throughout the upstream and downstream processes or in the final product (DS or DP). To date, there are no guidelines for acceptable doses of residual transfection reagent in virus preparations. However, the measurement of the residual rate seems to be obligatory to inform the regulatory production files and to have a traceabillity on the residual impurities in a viral preparation for therapeutic purposes which could represent a risk of toxicity.

An increasing demand for viral vectors is required to meet the expected need at the commercial level as well as the need generated by the rapid progression through the various phases of clinical development (*Van Der Loo-Human Molecular Genetics_2015*)*.* Consequently, the development of large-scale production, new producer cell lines, new synthetic media or new transfection reagents are under investigation to increase the viral vector productivity at the upstream level.

Recently, new transfection reagents based on heterocyclic compounds grafted to cationic polymers have shown improved production yields of viral vectors including AAV and lentiviruses when compared to production achieved with PEI, considered as the gold standard transfection reagent (WO2021/023796; WO2021/023798). However, the assay (sometimes designated "residual assay" or "residual test") for residual contents of these new transfection reagents in the yielded viral vectors or during the processes for their production is not yet available.

Thus the aim of the invention is to develop a specific method able to determine the amount of a material in a complex liquid mixture. This material is present in very low quantity in a liquid mixture which further comprises a biological matrix. The biological matrix comprises a recombinant virus and/or virus-like particles and may be a cell culture medium, a buffer or any solution used during both the upstream (UP) and downstream process generating at the end a final drug substance. The material, by definition a transfection reagent, is used to produce viral vector (AAV, LV, Adenovirus, oncolytic virus, baculovirus) in mammalian cells. Following the production of the viral vector (upstream process), several steps are required to eliminate all the impurity generated during the UP to finally obtain a pure drug active ingredient which could be administered to a patient.

Several residual tests have to be performed to ensure the patient safety. One of them concerns the transfection reagent. The transfection reagent contains usually a cationic polymer or lipid. Due to their cationic properties, these reagents have the capacity to interact with nucleic acids but also with many other chemicals (polymers, salts, small molecules, etc.) used in the whole production process of viral vectors.

To ensure that the level of these transfection reagents is restrained within safe amounts, the regulatory institutions in the field require the development of analytical methods able to detect and quantify such residuals.

The inventors had to consider several criteria to develop an accurate residual test:
- According to the biosafety level of the viruses, a preliminary virus inactivation step may be necessary to protect the manipulators. The inactivation step may be thermal (heating), chemical (detergents, acids, etc.) or physical (UV) and can generate by-products which could react with the transfection reagent. The choice of inactivation needs to be inefficient on the transfection reagent, i.e., needs to preserve the transfection reagent in the tested mixture that will be analyzed at the end.
- Moreover, as the transfection reagent could stick to or be encapsulated into the virus, in the assay for residual contents of the transfection reagent the virus should be fully degraded to give access to this potentially encapsulated reagent.
- At each step of the viral vector manufacturing process, the transfection reagent is present in a very low level compared to the other components (viruses, cell culture media, detergents, salts, etc.). The difficulty here is to detect this molecule into a complex liquid mixture. A specific purification step is needed to detect and quantify the residual transfection reagent. Considering this reagent is a macromolecule as are DNA, proteins of the virus, detergents used in the manufacturing or in the final formulation, a purification by size exclusion would not be accurate enough to allow a satisfying LOD (Limit of Detection) and LOQ (Limit of Quantification).
- The sensitivity of the analytical method requires a large range of detection capability, from 1 ppm (LOD) to 1000 ppm, which could correspond to the amount of transfection reagent in the concentration phase of the downstream process.

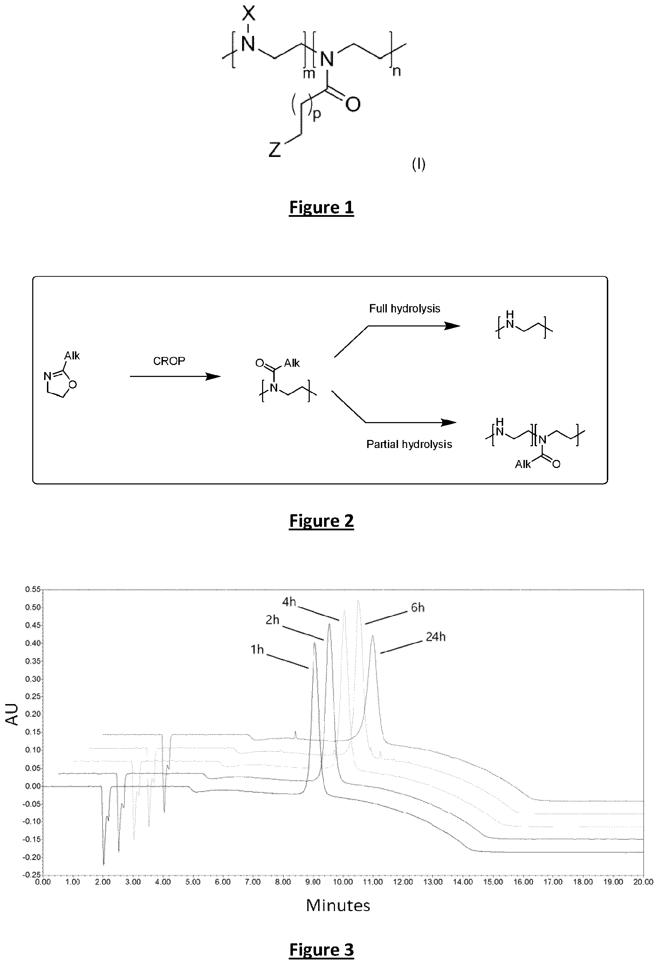

Because each viral vector manufacturing process is unique, it is difficult to anticipate the impact of a viral vector composition on the analytical test. To simplify and develop a residual test that would generalize to viral vector manufacturing, the inventors decided to focus their attention on the development of an orthogonal degradation which will not affect the transfection reagents of general formula (I) as described herein (**Figure 1**), which are well known for their transfection efficiency (Thomas Lorson, et al. Biomaterials 178 (2018) 204e280*; Ts.* Ivanova, E et al. PHARMACIA, 2016 vol. 63, 3*;* Nico Adams, et al. Reviews 59 (2007) 1504-1520*).*

PEI derivatives represent a large family of polymers used for nucleic acids delivery (DNA, siRNA, mRNA, miRNA, etc). In particular, linear PEIs (or IPEI) have shown strong transfection efficiency in both *in vitro* and *in vivo* applications. The synthesis of linear PEIs is based on a Cationic Ring Opening Polymerization (or CROP) of 2-alkyl-2-oxazolines generating polyalkyl-(2-oxazoline)s. Then, the polyalkyl-(2-oxazoline)s can be fully or partially hydrolyzed to generate IPEI or a combination of IPEI and polyalkyl-(2-oxazoline)s (Ryuichi Tanaka, et al. Macromolecules 1983, 16, 6, 849-853) (**Figure 2**).

Usually, the hydrolysis, which consists of a carbon-nitrogen bond cleavage of an amide's function, is performed under strong acidic conditions (Emi Haladjova, et al. Polymers 2020, 12, 2609*;* Alexander B. Cook, et al. Polym. Chem., 2019,10, 1202-1212*;* Emi Haladjova, et al. Macromol. Biosci. 2018, 1700349*).* Rangelov *et al.* performed partial degradation of poly(2-methyl-2-oxazoline) by an acidic hydrolysis at 100°C in an aqueous solution containing 17.5% of HCl (Haladjova E, et al. J Appl Polym Sci. 2020; e49400*;* R. Shah, et al., J. Mater. Sci. Mater. Med. 2015, 26, 157*).* Heating time and degree of hydrolysis were directly correlated. Between 15 to 180 min at 100°C, the degree of hydrolysis (DH) increased from 2-6% up to 60% (**Table 1**).

**Table 1. Influence of heating time.**

| **Polymer** | **k (s⁻¹)** | **DH for different time of hydrolysis (%)** | | | | |
|---|---|---|---|---|---|---|
| | | 15 min | 30 min | 60 min | 120 min | 180 min |
| PETOx-7.8 k | 6.8 x 10⁻⁵ | 6 | 10 | - | 38 | 53 |
| PETOx-9.6 k | 8.5 x 10⁻⁵ | 4 | 10 | 33 | 46 | 60 |
| PETOx-19.6 k | 5.9 x 10⁻⁵ | 2.5 | 4 | 14 | 30 | 59 |
| PETOx-Py-35.8 k | 7.5 x 10⁻⁵ | 2.6 | 6 | 14 | 41 | 60 |

Park *et al.* carried out at 100°C for 6h partial degradation of poly(2-ethyl-2-oxazoline) with various concentration of HCl (Ji Hoon Jeong, et al. Journal of Controlled Release 73 (2001) 391-399*;* R. Tanaka, et al., Macromolecules 16 (1983) 849-853*).* In this study, different concentrations of 5.0, 7.5, and 10.0% (v/v) HCl were used for the hydrolysis. As the concentration of HCl increased, the extent of hydrolysis increased as listed in **Table 2.**

**Table 2. Influence of HCl concentration.**

| **Acid hydrolysis of poly(2-ethyl-2-oxazoline) at 100°C** | | | |
|---|---|---|---|
| **Code** | **MW of poly(2-ethyl-2-oxazoline)** | **HCl concentration (%, v/v)** | **Percent hydrolysis^{a}** |
| A | 50 000 | 5.0 | 52.6 |
| B | 50 000 | 7.5 | 73.3 |
| C | 50 000 | 10.0 | 88.0 |
| D | 200 000 | 10.0 | 91.6 |

| | | | |
|---|---|---|---|
| ^{a} **Determined by using 400-MHz H NMR spectra.** | | | |

Knowing that similar acid conditions can be applied to the degradation of nucleic acids or proteins or main components of viral vectors, the inventors have studied the conditions of degradation by acidic hydrolysis of the viral vectors contained in a biological matrix made up of various components present during the manufacture, purification and storage of viral vectors. Several parameters have been studied such as the source and concentration of acid, temperature, heating time to design a general method capable of specifically degrading a viral vector or any amide function except those included in the transfection reagents based on the general formula (**I**) (**Figure 1**).

The inventors provide a test able to detect and quantify PEI-based transfection reagents in a liquid mixture which comprises a biological matrix comprising recombinant viruses or virus-like particles and including the following steps:
- Optionally a viral vector inactivation,
- A specific acidic hydrolysis, which degrades a viral vector or any amide function except thoses of the PEI-based transfection reagents of general formula, *i.e.*, said specific acidic hydrolysis does not affect PEI-based transfection reagents,
- A purification step able to separate PEI-based transfection reagents from by-products generated by the acidic hydrolysis,
- A qualitative and quantitative analysis of the residual PEI-based transfection reagents.

The method developed by the inventors shows several benefits. The method includes a purification of the transfection reagent meaning the analysis is specific, highly sensitive, robust and not dependent on sample composition. The method is applicable to different virus types or subtypes, at various concentrations and fully independent of the virus biosafety level. The method is not to be modified by the biological matrix nature (buffer, detergent, pH, etc.). The method is easily handled and may be used for research and development, clinical or commercial samples. The method may be GMP (good manufacturing practice) grade qualified.

Thus it is an object of the present invention to provide a method for performing an acidic hydrolysis of a liquid mixture comprising a biological matrix and a polyethyleneimine (PEI)-based transfection reagent,
wherein the biological matrix comprises a recombinant virus or virus-like particles produced using the PEI-based transfection reagent,
wherein the method comprises the step of incubating the liquid mixture comprising the biological matrix in an aqueous solution comprising from 0.1% to 10% (v/v) hydrochloric acid (HCl) at a temperature ranging from 60°C to 110°C for a time period ranging from 2 hours to 24 hours, preferably an aqueous solution comprising 0.1% (v/v) HCl at a temperature of 110°C for 2 hours or in an aqueous solution comprising 1% (v/v) HCl at a temperature ranging from 60°C to 80°C for 2 hours,
wherein said acidic hydrolysis does not degrade the PEI-based transfection reagent, and wherein the PEI-based transfection reagent is of general formula (I) or an acceptable salt thereof: 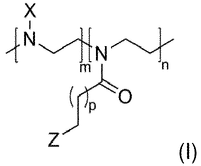
wherein:
   - m represents an integer between 27 to 1200, preferably an integer between 200 to 600, and n represents an integer between 3 to 600, preferably an integer between 20 to 300, with the proviso that n is lower than m and the sum of m+n ranges from 30 to 1200,
   - X represents H or a group of formula: 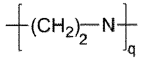 in which q represents an integer between 10 and 800, preferably 20 and 400,
   - p represents an integer between 1 and 4,
   - Z represents a group of formula: 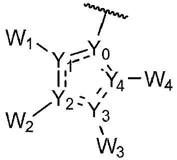
   - Y₀, Y₁, Y₂, Y₃ and Y₄, which may be identical or different, represent C or N, with the proviso that at least two, but no more than three, of Y₀, Y₁, Y₂, Y₃ and Y₄, are N,
   - W₁, W₂, W₃ and W₄, which may be identical or different, represent H, a linear or branched, saturated or unsaturated C₁-C₁₈ alkyl, C₆-C₁₈ aryl, a linear or branched, saturated or unsaturated C₆-C₁₈ aryl-C₁-C₁₈ alkyl, C₅-C₁₀ heteroaryl, a linear or branched, saturated or unsaturated C₂-C₁₈ heteroalkyl, an amine, a linear or branched, saturated or unsaturated C₁-C₁₈ alkylamine, a C₁₋C₁₂ alkoxy; or (i) W₁ and W₂ or (ii) W₂ and W₃ or (iii) W₃ and W₄ together form a fused, optionally substituted six-membered aryl; or a fused, optionally substituted six-membered heteroaryl containing no more than 1 N atom,
with the proviso that at least one, but no more than two, of W₁, W₂, W₃ and W₄ is, or are, absent.

As defined herein, the term *"C₁-C₁₈ alkyl"* represents any monovalent radical of a linear or branched hydrocarbon chain comprising 1 to 18 carbon atoms. Examples of suitable C₁-C₁₈ alkyl groups include, but are not limited to, C₁-C₄ alkyl groups such as methyl, ethyl, n-propyl, i-propyl, n-butyl, i-butyl, s-butyl or t-butyl, C₆-C₈ alkyl groups such as n-hexyl, n-heptyl or n-octyl, as well as n-pentyl, 2-ethylhexyl, 3,5,5-trimethylhexyl, n-nonyl, n-decyl, n-undecyl, n-dodecyl or *n-*octadecyl.

As defined herein, the term *"C₆-C₁₈* aryl" represents any monovalent radical of an aromatic hydrocarbon comprising 6 to 18 carbon atoms. Examples of suitable C₆-C₁₈ aryl groups include, but are not limited to, phenyl, naphthyl, anthracenyl or phenanthrenyl.

As defined herein, the term "*C₆-C₁₈ aryl-C₁-C₁₈ alkyl*" represents an aryl group as defined herein combined to an alkyl group as defined herein. Examples of suitable C₆-C₁₈ aryl-C₁-C₁₈ alkyl groups include, but are not limited to, benzyl, phenylethyl (or phenethyl), phenylpropyl, phenylbutyl, phenylpentyl, phenylhexyl, naphthylmethyl, naphthylethyl, naphthylpropyl, naphthylbutyl, naphthylpentyl, naphthylhexyl, anthracenylmethyl, anthracenylethyl, anthracenylpropyl, anthracenylbutyl, anthracenylpentyl, anthracenylhexyl, phenanthrenylmethyl, phenanthrenylethyl, phenanthrenylpropyl, phenanthrenylbutyl, phenanthrenylpentyl or phenanthrenylhexyl.

As defined herein, the term "*C₅-C₁₀ heteroaryl*" represents any monovalent radical of a monocyclic or bicyclic 5 to 10 membered aromatic group comprising from 1 to 3 heteroatoms independently selected from oxygen, nitrogen and sulfur. Examples of suitable C₅-C₁₀ heteroaryl groups include, but are not limited to, furyl, thienyl, pyrrolyl, pyrazoyl, imidazolyl, isoxazolyl, isothiazoyl, thiazolyl, oxazolyl, 1,2,3-triazolyl, 1,2,4-triazolyl, 1-benzofuryl, 1-benzothienyl, indolyl, benzimidazolyl, indazolyl, 1,2-benzisoxazolyl, 2,1-benzisoxazolyl, 1,2-benzisothiazolyl, 2,1-benzisothiazolyl, benzothiazolyl, benzoxazolyl, benzotriazolyl, pyridyl, pyridinium, quinolinyl, quinolinium, isoquinolinyl, isoquinolinium, pyridazinyl, cinnolinyl, phthalazinyl, pyrimidinyl, quinazolinyl, pyrazinyl or quinoxalinyl.

As defined herein, the term "*C₂-C₁₈ heteroalkyl*" represents an alkyl group as defined herein substituted by one or more heteroatoms such as O, N, or S.

As defined herein, the term "*C₁₋C₁₈ alkylamine*" represents any monovalent radical of a linear or branched hydrocarbon chain comprising 1 to 18 carbon atoms, in which one of the hydrogen atom bonded to a carbon atom is replaced by an amino group. Examples of suitable C₁-C₁₈ alkylamine include, but are not limited to, -(CH₂)ₙ-NH₂, with n representing an integer between 1 and 18, -CH₂NHCH₃, -CH₂CH(CH₃)-NH₂, or -(CH₂)ₙ N(CH₃)₂, with n representing an integer between 1 and 6.

As defined herein, the term "*C₁₋C₁₂ alkoxy*" represents a radical of formula -OR', wherein R' is a C₁-C₁₂ alkyl. Examples of suitable C₁-C₁₂ alkoxy groups include, but are not limited to, C₁-C₆ alkoxy groups such as methoxy (-OCH₃), ethoxy (-OCH₂CH₃), t-butoxy (-OC(CH₃)₃), or - O(CH₂)₅CH₃.

Unless mentioned otherwise, the groups and radicals defined hereinabove may be unsubstituted or substituted by one or more substituents such as, for example, halogen, alkyl, alkoxy, aryl, heteroaryl, haloalkyl, haloalkoxy, alkoxycarbonyl, alkanoyl, aroyl, formyl, nitrile, nitro, amido, alkylthio, alkylsulfinyl, alkylsulfonyl, arylthio, arylsulfinyl, arylsulfonyl, amino, alkylamino, arylamino, dialkylamino and diarylamino.

As defined herein, the term "*halogen*" represents an atom of F, Cl, Br or I.

In a particular embodiment of the invention, the PEI-based transfection reagent of general formula (I) as defined herein is one wherein W₁, W₂, W₃ and W₄, which may be identical or different, represent H, methyl, cyclopropyl, isopropyl, tert-butyl, phenyl, benzyl, 2-pyridine, 3-pyridine, or 4-hydroxyphenethyl; or wherein (i) W₁ and W₂ or (ii) W₂ and W₃ or (iii) W₃ and W₄ together form a fused phenyl; a fused phenyl substituted by a methyl group, in particular two methyl groups, a methoxy group, a carboxyphenyl or Cl; a fused naphthalene; a fused 2-pyridine; or a fused 3-pyridine.

In a particular embodiment of the invention, the PEI-based transfection reagent of general formula (I) as defined herein is one wherein X represents a branched PEI, which is a group of formula: 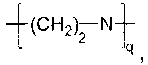 in which q represents an integer between 10 and 800, preferably 20 and 400.

Examples of branched PEIs are well known to the person skilled in the art.

In a preferred embodiment of the invention, the PEI-based transfection reagent of general formula (I) as defined herein is one wherein X represents H, *i.e.* X is a linear PEI. Examples of linear PEIs (IPEIs) are well known to the person skilled in the art.

In a particular embodiment of the invention, the PEI-based transfection reagent of general formula (I) as defined herein has a grafting ratio defined as (n/(m+n))*100, wherein the grafting ratio is ranging from 1 to 50%, preferably from 5 to 30%, more preferably is 20%.

As defined herein, the term "*grafting ratio*" refers to the number of grafted monomer units on primary or secondary amino groups by side chains, divided by the number of total monomer units present in an original cationic polymer backbone (*i.e.,* the PEI polymer backbone). The grafting ratio will depend upon the molecular weight of the cationic polymer, the chemical reactivity of the grafted side chains onto the polymer, or the obtained biological effect. Said grafting ratio may be determined by a measurement method well known in the art, for exemple by NMR.

The PEI polymer backbone is of general formula (II): 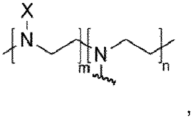 wherein ₘ, ₙ and X are as defined herein.

In a particular embodiment of the invention, the PEI polymer backbone has an average molecular weight (Mw) ranging from 1 kDa to 50 kDa, preferably from 5 kDa to 30 kDa or from 10 kDa to 25 kDa, more preferably the PEI polymer backbone has an average molecular weight (Mw) of 8, 10, 15, 22, 25 or 30 kDa, preferably of 22 kDa.

In a particular embodiment of the invention, in the general formula (I) of the PEI-based transfection reagent, the sum of m+n ranges from 30 to 1200, preferably from 200 to 600, and the average molecular weight (Mw) of the PEI polymer backbone ranges from 1 kDa to 200 kDa, in particular from 1 kDa to 50 kDa, preferably from 5 kDa to 30 kDa or from 10 kDa to 25 kDa, more preferably is of 8, 10, 15, 22, 25 or 30 kDa, even more preferably of 22 kDa.

In a particular embodiment of the invention, m represents an integer between 27 to 600 and n represents an integer between 3 to 600. Preferably, m represents an integer between 198 to 300 and n represents an integer between 2 to 300.

In another particular embodiment of the invention, the sum of m+n ranges from 30 to 1200, preferably from 200 to 600.

In a preferred embodiment of the invention, the PEI polymer backbone has an average molecular weight (Mw) of 8, 10, 15, 22, 25 or 30 kDa, preferably of 22 kDa. When the average molecular weight (Mw) of the PEI polymer backbone is 8, 10, 15, 22, 25 or 30 kDa, the sum of m+n is equal to 180, 220, 340, 500, 570 or 680 respectively.

In preferred embodiment of the invention, the PEI-based transfection reagent of general formula (I) is selected from the group consisting of the following compounds:

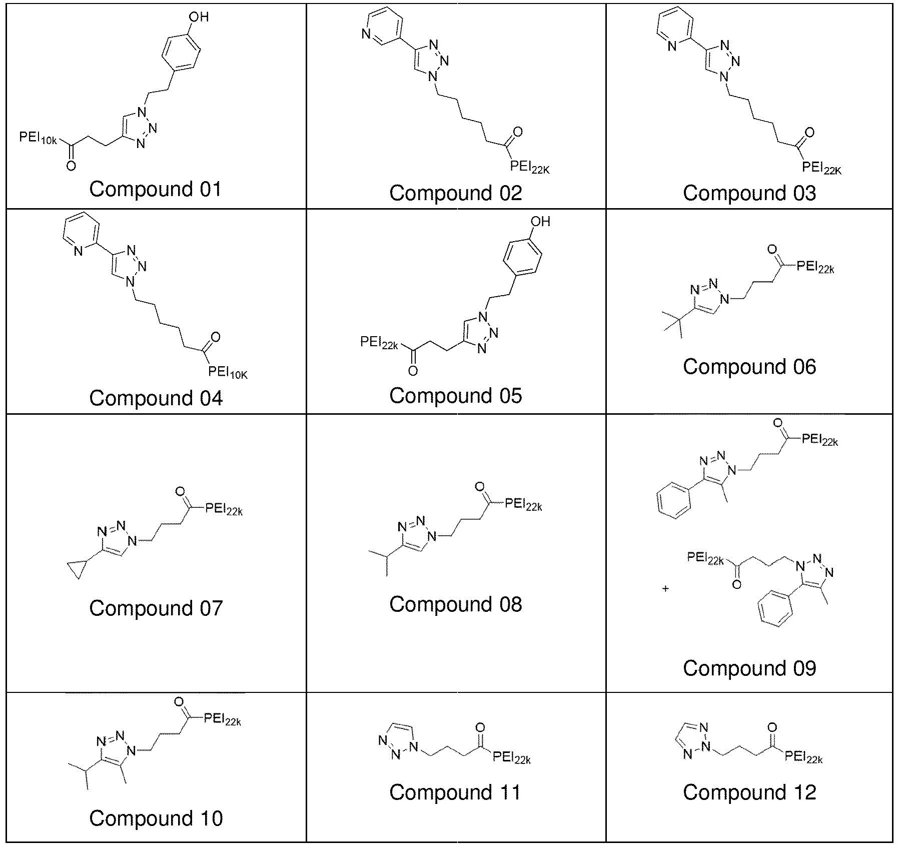

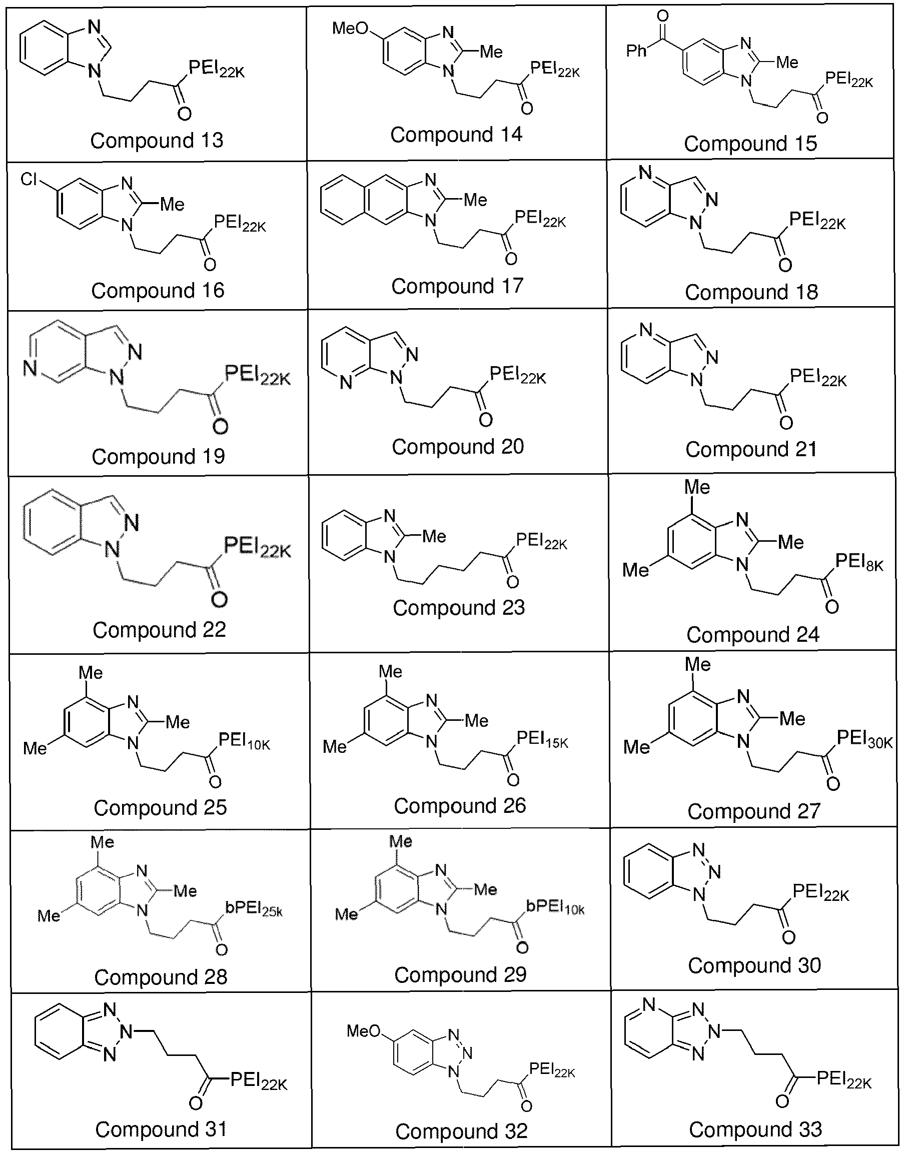

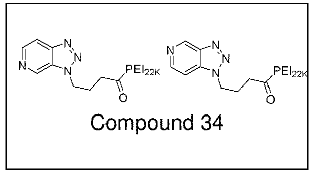

In these compounds 1-34, the term "*PEI*" refers to a linear PEI, and the term "*bPEI*" refers to a branched PEI.

The compound of general formula (I) may be prepared according to various methods well known in the art, for example as disclosed in the patent applications WO2021/023796 and WO2021/023798.

In a particular embodiment of the invention, the biological matrix is selected from the group consisting of a cell culture medium, in particular a culture medium of eukaryotic cells, in particular suspension cells or adherent cells, a buffer, a solution used during the manufacturing and purification process of recombinant viruses, and a final composition comprising the purified viruses in a final formulation comprising pharmaceutically acceptable buffer and excipients.

As defined herein, the term "*cell culture medium*" has the meaning known in the art and refers for example to a medium containing at least one of the following components: serum, synthetic medium, animal-free component medium or chemically defined medium, in particular medium for maintaining cells alive, or for growing, for differentiating or for expanding cells, or for enhancing transfection.

As defined herein, the term "*suspension cells*" refers to cells that do not need solid support for growth and are thus anchorage-independent. Examples of suspension cells include, but are not limited to, NSO cells, U937 cells, Namalawa cells, HL60 cells, WEHI231 cells, Yac 1 cells, Jurkat cells, THP-1 cells, K562 cells or U266B1 cells.

As defined herein, the term "*adherent cells*" refers to cells that need solid support for growth and are thus anchorage-dependent. Examples of adherent cells include, but are not limited to, MRC-5 cells, HeLa cells, Vero cells, NIH-3T3 cells, L293 cells, CHO cells, BHK-21 cells, MCF-7 cells, A549 cells, COS cells, HEK 293 cells, Hep G2 cells, SNN-BE(2) cells, BAE-1 cells or SH-SY5Y cells.

As defined herein, the term "*buffer*" refers to a buffer solution comprising a buffering agent. As defined herein, the term "*buffering agent*" refers to an agent that adjusts, maintains or controls the pH of a solution. Buffering agents can be either the weak acid or weak base that would comprise a buffer solution. Examples of suitable buffering agents include, but are not limited to, sodium carbonate, sodium bicarbonate, sodium hydroxide, calcium bicarbonate, calcium citrate, sodium citrate, magnesium hydroxide, magnesium bicarbonate, potassium acetate, Tris acetate, sodium acetate, potassium phosphate monobasic, potassium carbonate, potassium bicarbonate, potassium citrate, or magnesium oxide.

As defined herein, the expression "*a solution used during the manufacturing and purification process of recombinant viruses*" refers to any solution well known in the art that can be used during the manufacturing and purification process of recombinant viruses.

As defined herein, the term "*pharmaceutically acceptable buffer and excipients*" refers to a pharmaceutically acceptable vehicle, which is any substance or combination of substances physiologically acceptable *i.e.*, appropriate for its use in a composition in contact with a host (especially for administration to a host), especially a human, and thus non-toxic. It can refer to a solid, semi-solid or liquid filler, diluent, encapsulating material or formulation auxiliary of any conventional type. Examples of suitable acceptable excipients include, but are not limited to, glucose, galactose, lactose, dextrose, maltose, mannitol, sucrose, trehalose, polyethyleneglycol, or pluronic acid.

It is another object of the present invention to provide a method for purifying, detecting and/or quantifying a polyethyleneimine (PEI)-based transfection reagent of general formula (I) as defined herein, wherein the PEI-based transfection reagent of general formula (I) is comprised in a liquid mixture comprising a biological matrix,
wherein the biological matrix comprises a recombinant virus or virus-like particles produced using the PEI-based transfection reagent of general formula (I),
wherein the method comprises the steps of:
   (a) performing an acidic hydrolysis of the liquid mixture according to the method of the invention,
   (b) purifying the reaction mixture obtained in step (a) in order to obtain a purified PEI-based transfection reagent of general formula (I),
   (c) detecting and/or quantifying the purified PEI-based transfection reagent of general formula (I) obtained in step (b).

As defined herein, the term "*purifying*" means that other constituents of the solution are separated or removed to keep the solution of the transfection reagent only.

In a particular embodiment of the invention, before performing step (a), the method comprises a step of inactivating the recombinant virus or virus-like particles by heating the biological matrix at a temperature ranging from 110°C to 130°C for a time period ranging from 30 minutes to 4 hours, preferably at a temperature of 120°C for 30 minutes.

In a particular embodiment of the invention, step (b) is performed using ultrafiltration or centrifugation.

In a particular embodiment of the invention, step (c) is performed using High-performance liquid chromatography (HPLC) or Ultra high performance liquid chromatography (UHPLC) analytical technique, preferably UHPLC.

In a particular embodiment of the invention, the PEI-based transfection reagent of general formula (I) of step (c) is detected with a limit of detection (LOD) ranging from 1 ppm to 1000 ppm, and/or a limit of quantification (LOQ) ranging from 1 ppm to 1000 ppm.

In a particular embodiment of the invention, the PEI-based transfection reagent of general formula (I) is detectable in a biological matrix during the manufacturing process of the recombinant viruses, wherein the biological matrix is selected from the group consisting of a cell culture medium, in particular a culture medium of eukaryotic cells, in particular suspension cells or adherent cells, a buffer, a solution used during the manufacturing and purification process of recombinant viruses, and a final composition comprising purified viruses in a final formulation comprising pharmaceutically acceptable buffer and excipients.

In another particular embodiment of the invention, the PEI-based transfection reagent is used during the manufacturing process of an advanced therapy medicinal product (ATMP) and is present in residual quantity (1 to 1000 ppm) with respect to transfection reagents provided in the manufacturing process and to other components of the liquid mixture.

The terms "*cell culture medium*", "*suspension cells*", "*adherent cells*", "*buffer*", "*a solution used during the manufacturing and purification process of recombinant viruses*" and "*pharmaceutically acceptable buffer and excipients*" are defined according to the above definitions and in accordance with examples provided herein.

In a particular embodiment of the invention, the recombinant virus is selected from the group consisting of an adeno-associated virus (AAV), a lentivirus (LV), an adenovirus, an oncolytic virus and a baculovirus, preferably is an adeno-associated virus (AAV) or a lentivirus (LV), more preferably is an adeno-associated virus (AAV).

In a particular embodiment of the invention, the PEI-based transfection reagent of general formula (I) is a compound selected from the group consisting of compounds 01, 02, 03, 04, 05, 06, 07, 08, 09,10,11,12, 30, 31, 32, 33 and 34, and the recombinant virus is an adeno-associated virus (AAV).

Other features and advantages of the invention will be apparent from the examples which follow and will also be illustrated in the figures.

### BRIEF DESCRIPTION OF THE FIGURES

**Figure 1****. Transfection reagent of general formula (I).**
**Figure 2****. Synthesis of PEl by CROP.**
**Figure 3****. Optimization of thermal inactivation with compound 05.**
**Figure 4. Figure 4a****. Hydrolysis of compound 05.** **Figure 4b****. Overlay of** **Figure 4a****.**
**Figure 5****. Determination of the linearity of the hydrolysis of compound 05.**

### EXAMPLES

### Example 1. General procedure for the preparation of grafted polymers

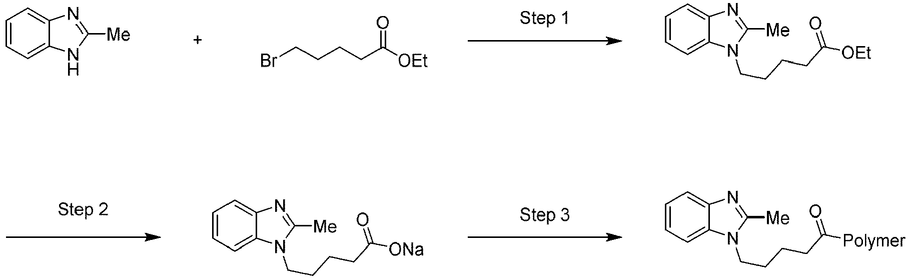

### Step 1: N-alkylation of heterocycles

In an oven-dried round-bottom flask under argon was added the corresponding heterocycle (1 equiv.) and DMF (2 ml/mmol of starting material). The solution was cooled to 0°C and Sodium Hydride (60% dispersion in mineral oil, 1.2 equiv.) was added by portion. The mixture was slowly warmed up to room temperature over 1 hour. Then, the corresponding ester was added dropwise and the reaction was stirred at room temperature for 4-12 hours. The mixture was quenched by addition of water (10 mL/1 mL of DMF) and the aqueous layer was extracted with EtOAc. (5 × 2 mL/ 1 mL of DMF). The combined organic extracts were washed with brine and dried over anhydrous MgSO₄. After filtration, the solvent was removed in vacuo and the resulting oil was purified by column chromatography (EtOAc 20 to 50% in heptane).

### Step 2: Saponification of acid moieties

To a solution of ester in EtOH (2 mL/mmol of ester) was added dropwise a 5 M solution of NaOH (0.2 mL/mmol of ester), and the mixture was stirred at room temperature overnight. Then, the solvent was removed in vacuo and the residue was purified by column chromatography on SiO₂ using MeOH 5% in DCM + AcOH 1% or using Acetonitrile 0 to 100 % in H₂O.

### Step 3: Grafting

In a round-bottom flask was added the cationic polymer (1 equiv.) in water (4 mL/mmol of starting material) followed by N-methyl morpholine or NMM (2 equiv.). The carboxylate (0.3-1 equiv.) was added followed by MeOH (16 mL/mmol of polymer). After stirring 10 minutes, 4-(4,6-dimethoxy-1,3,5-triazin-2-yl)-4-methylmorpholinium chloride was added or DMTMM (0.6-2 equiv.) and the mixture was stirred 12-24 hours at room temperature. Then, MeOH was removed in vacuo, water (4 mL/mmol of starting material) followed by a solution of 3M HCl (1 mL/mmol of starting material) were added. The residue was purified using a dialysis cassette in a 50 mM HCl bath.

### Step 4: Synthesis of triazole by « click » chemistry starting from an acid.

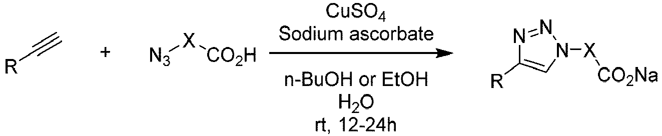

or

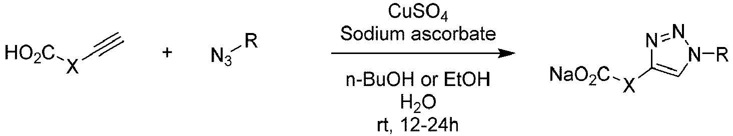

Alkyne (1 equiv.), azide (1 equiv.), CuSO₄ (0.01 equiv) and sodium ascorbate (0.03 equiv) were added to a 2:1 (v/v) solution of nBuOH and water. The reaction was stirred at room temperature for 24 hours. Then, NaOH (5 M, 2 equiv.) was added, and the organic solvent was removed in vacuo. The residue was purified by reversed phase flash chromatography using 0 to 100% CH₃CN in water as eluant.

### Step 5: Synthesis of triazole by « click » chemistry starting from an ester

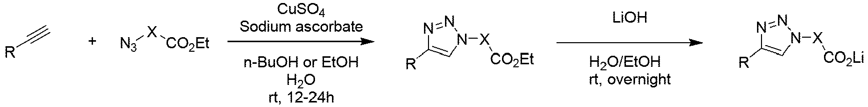

or

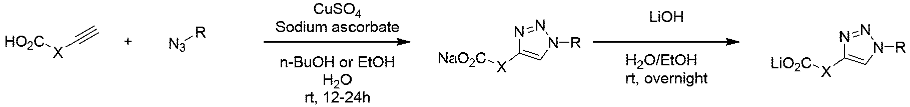

Alkyne (1 equiv.), azide (1 equiv.), CuSO₄ (0.01 equiv) and sodium ascorbate (0.03 equiv) were added to a 2:1 (v/v) solution of nBuOH and water. The reaction was stirred at room temperature for 24 hours. Then, NaOH (5 M, 2 equiv.) was added, and the organic solvent was removed in vacuo. The residue was purified by reversed phase flash chromatography using 0 to 100% CH₃CN in water as eluant.

### Step 6: Saponification of the ester moiety.

To a solution of ester in EtOH was added dropwise a 3 M solution of LiOH, and the mixture was stirred at rt for the weekend. Then, the solvent was removed in vacuo and the residue was purified by reverse phase FC on SiO₂ using H₂O/MeCN as eluant using a Biotage Flash purification system. The acid obtained was lyophilized to yield a solid.

### Example 2. Syntheses of PEI-based transfection reagents of the invention

### - Synthesis of compound 13 (Polymer Molecular weight = 22k, Heterocycle grafting = 10%)

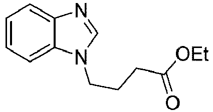

Intermediate **13.a** was prepared analogously to the general procedure, step 1 (Example 1). Yield= 60% ; m= 2.30 g ; ¹H NMR (400 MHz, Chloroform-*d*) δ 7.94 (s, 1H), 7.81 - 7.72 (m, 1H), 7.43 - 7.36 (m, 1H), 7.26 (ddd, *J* = 13.1, 7.5, 4.9 Hz, 2H), 4.24 (t, *J* = 7.0 Hz, 2H), 4.09 (q, *J* = 7.4 Hz, 2H), 2.28 (t, *J* = 7.0 Hz, 2H), 2.16 (p, *J* = 7.0 Hz, 2H), 1.20 (t, *J* = 7.1 Hz, 3H).

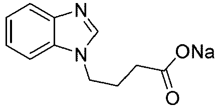

Intermediate **13.b** was prepared analogously to the general procedure, step 2 (Example 1). Yield= 45% ; m= 1.00 g ; ¹H NMR (400 MHz, Methanol-*d*₄) δ 8.21 (d, *J* = 2.4 Hz, 1H), 7.70 (d, *J* = 8.0 Hz, 1H), 7.63 (d, *J* = 8.0 Hz, 1H), 7.33 (dt, *J* = 17.3, 7.5 Hz, 2H), 4.37 (t, *J* = 7.4 Hz, 2H), 2.35 (t, *J* = 7.4 Hz, 2H), 2.18 (p, *J* = 7.4 Hz, 2H).

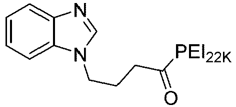

Compound **13** was prepared analogously to the general procedure, step 3 (Example 1). Yield= 94% ; m= 117 mg ; ¹H NMR (D₂O) δ: ¹H NMR (400 MHz, Deuterium Oxide) δ 9.29 - 8.97 (m, 1H), 7.96 - 7.17 (m, 4H), 4.46 (d, *J* = 42.4 Hz, 2H), 3.45 (s, 39H), 2.35 (dd, *J* = 135.3, 58.1 Hz, 4H).

### - Synthesis of compound 14 (Polymer Molecular weight = 22k, Heterocycle grafting = 24%)

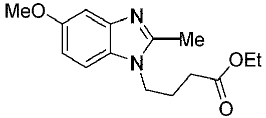

Intermediate **14.a** was prepared analogously to the general procedure, step 1 (Example 1). Yield= 63%; m= 1.7 g; ¹H NMR (400 MHz, Chloroform-*d*) δ 7.63 (d, *J* = 8.7 Hz, 0H), 7.27 (d, *J* = 8.8 Hz, 1H), 6.99 - 6.86 (m, 2H), 4.27 - 4.15 (m, 4H), 3.93 (dd, *J* = 7.3, 1.3 Hz, 3H), 2.65 (dd, *J* = 3.7, 1.3 Hz, 3H), 2.43 (q, *J* = 6.8 Hz, 2H), 2.18 (p, *J* = 7.1 Hz, 2H), 1.33 (td, *J* = 7.2, 1.3 Hz, 3H).

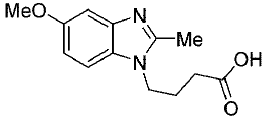

Intermediate **14.b** was prepared analogously to the general procedure, step 2 (Example 1). Yield= 100%; m= 676 mg; ¹H NMR (400 MHz, Methanol-*d*₄) δ 7.54 (dd, *J* = 32.8, 8.9 Hz, 1H), 7.18 (dd, *J* = 33.3, 2.4 Hz, 1H), 7.01 (ddd, *J* = 21.3, 8.9, 2.3 Hz, 1H), 4.33 (q, *J* = 6.8, 6.3 Hz, 2H), 3.92 - 3.84 (m, 3H), 2.70 (d, *J* = 8.0 Hz, 3H), 2.43 (q, *J* = 6.7 Hz, 2H), 2.12 (p, *J* = 7.0 Hz, 2H).

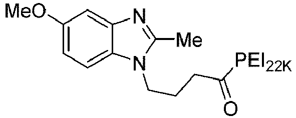

Compound **14** was prepared analogously to the general procedure, step 3 (Example 1). Yield= 100%; m= 175 mg; ¹H NMR (400 MHz, Deuterium Oxide) δ 7.80 - 6.57 (m, 3H), 4.44 - 2.96 (m, 22H), 2.80 - 1.38 (m, 7H).

### - Synthesis of compound 15 (Polymer Molecular weight = 22k, Heterocycle grafting = 27%)

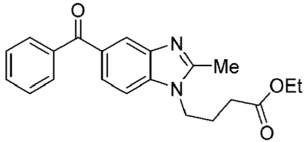

Intermediate **15a** was prepared analogously to the general procedure, step 1 (Example 1). Yield= 50%; m= 820 mg; ¹H NMR (400 MHz, Chloroform-d) δ 7.94 - 7.21 (m, 8H), 7.05 (d, *J* = 1.3 Hz, 1H), 5.08 (d, *J* = 1.3 Hz, 2H), 4.05 (td, *J* = 8.1, 7.5, 2.9 Hz, 2H), 3.92 (dtd, *J* = 16.2, 7.8, 6.5 Hz, 2H), 2.50 (dd, *J* = 12.5, 1.4 Hz, 3H), 2.19 (q, *J* = 6.3 Hz, 2H), 1.94 (p, *J* = 7.0 Hz, 2H), 1.04 (ddd, *J* = 14.3, 7.9, 6.5 Hz, 3H).

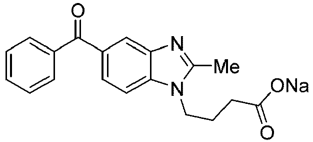

Intermediate **15.b** was prepared analogously to the general procedure, step 2 (Example 1). Yield= 73%; m= 580 mg; ¹H NMR (400 MHz, Methanol-*d*₄) δ 8.01 (d, *J* = 8.9 Hz, 1H), 7.83 - 7.48 (m, 7H), 4.34 (tt, *J* = 7.3, 3.0 Hz, 2H), 2.71 - 2.65 (m, 2H), 2.41 (ddt, *J* = 10.2, 7.4, 4.2 Hz, 2H), 2.12 (h, *J* = 7.2 Hz, 2H).

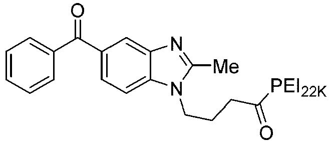

Compound **15** was prepared analogously to the general procedure, step 3 (Example 1). Yield= 84%; m= 171 mg; ¹H NMR (400 MHz, Deuterium Oxide) δ 7.92 - 6.49 (m, 8H), 4.50 - 3.10 (m, 17H), 3.03 -1.78 (m, 7H).

### - Synthesis of compound 16 (Polymer Molecular weight = 22k, Heterocycle grafting = 30%)

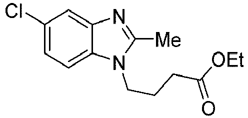

Intermediate **16a** was prepared analogously to the general procedure, step 1 (Example 1). Yield= 91%; m= 1.55 g; ¹H NMR (400 MHz, Chloroform-d) δ 7.68 - 7.53 (m, 1H), 7.33 - 7.16 (m, 2H), 4.14 (pd, *J* = 7.4, 1.6 Hz, 4H), 2.61 (d, *J* = 1.5 Hz, 3H), 2.35 (td, *J* = 6.9, 3.5 Hz, 2H), 2.09 (h, *J* = 5.8, 4.7 Hz, 2H), 1.26 (tdd, *J* = 6.9, 4.5, 1.5 Hz, 3H).

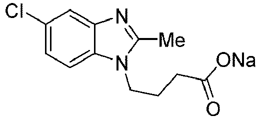

Intermediate **16b** was prepared analogously to the general procedure, step 2 (Example 1). Yield= 60%; m= 840 mg; ¹H NMR (400 MHz, Methanol-*d*₄) δ 7.85 (s, 1H), 7.75 - 7.60 (m, 1H), 7.45 - 7.38 (m, 1H), 4.39 (q, *J* = 7.6, 6.5 Hz, 2H), 2.77 (t, *J* = 2.9 Hz, 3H), 2.49 (t, *J* = 6.6 Hz, 2H), 2.13 (p, *J* = 7.3 Hz, 2H).

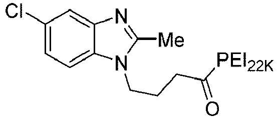

Compound **16** was prepared analogously to the general procedure, step 3 (Example 1). Yield= 100%; m= 193 mg; ¹H NMR (400 MHz, Deuterium Oxide) δ 7.83 - 6.65 (m, 3H), 4.52 - 3.09 (m, 15H), 3.04 - 2.32 (m, 5H), 2.30 - 1.72 (m, 2H).

### - Synthesis of compound 17 (Polymer Molecular weight = 22k, Heterocycle grafting = 26%)

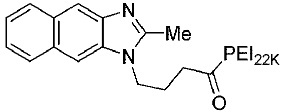

Compound **17** was prepared analogously to the general procedure, step 3 (Example 1). Yield= 97%; m= 198 mg; ¹H NMR (400 MHz, Deuterium Oxide) δ 8.19 - 6.14 (m, 6H), 4.51 - 0.73 (m, 24H).

### - Synthesis of compound 18 (Polymer Molecular weight = 22k, Heterocycle grafting = 47%)

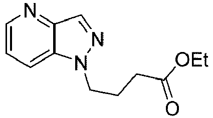

Intermediate 18a was prepared analogously to the general procedure, step 1 (Example 1). Yield= 15%; m= 211 mg; ¹H NMR (400 MHz, Chloroform-d) δ 8.52 (dd, *J* = 4.4, 1.3 Hz, 1H), 8.19 (d, *J* = 1.0 Hz, 1H), 7.77 (dt, *J* = 8.5, 1.2 Hz, 1H), 7.24 (dd, *J* = 8.6, 4.4 Hz, 1H), 4.46 - 4.38 (m, 2H), 4.04 (q, *J* = 7.2 Hz, 2H), 2.28 - 2.13 (m, 4H), 1.17 (t*, J* = 7.1 Hz, 3H).

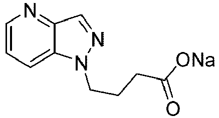

Intermediate **18b** was prepared analogously to the general procedure, step 2 (Example 1). Yield= 100%; m= 887 mg; ¹H NMR (400 MHz, Deuterium Oxide) δ 8.24 (dd, *J* = 4.5, 1.3 Hz, 1H), 7.92 (d, *J* = 1.0 Hz, 1H), 7.76 (dt, *J* = 8.7, 1.2 Hz, 1H), 7.20 (dd, *J* = 8.7, 4.4 Hz, 1H), 4.21 - 4.13 (m, 2H), 2.02 - 1.87 (m, 4H).

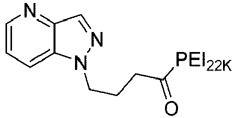

**Compound 18** was prepared analogously to the general procedure, step 3 (Example 1). Yield= 97 %; m= 103 mg; ¹H NMR (400 MHz, Deuterium Oxide) δ 8.92 - 7.42 (m, 4H), 4.61 - 4.25 (m, 2H), 4.04 - 3.09 (m, 10H), 2.67 - 1.88 (m, 5H).

### - Synthesis of compound 19 (Polymer Molecular weight = 22k, Heterocycle grafting = 25%)

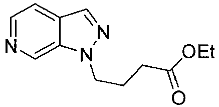

Intermediate **19a** was prepared analogously to the general procedure, step 1 (Example 1). Yield= 43%; m= 602 mg; ¹H NMR (400 MHz, Chloroform-*d*) δ 9.00 (s, 1H), 8.29 (d, *J* = 5.6 Hz, 1H), 8.04 (d, *J* = 0.8 Hz, 1H), 7.61 (dd, *J* = 5.6, 1.3 Hz, 1 H), 4.61 - 4.52 (m, 2H), 4.09 (q, *J* = 7.1 Hz, 2H), 2.34 - 2.21 (m, 4H), 1.20 (t, *J* = 7.1 Hz, 3H).

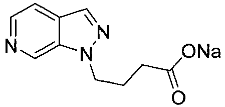

Intermediate **19b** was prepared analogously to the general procedure, step 2 (Example 1). Yield=100 %; m= 592 mg; ¹H NMR (400 MHz, Deuterium Oxide) δ 8.77 (s, 1H), 8.04 - 7.93 (m, 2H), 7.57 (dd, *J* = 5.8, 1.3 Hz, 1H), 4.37 - 4.29 (m, 2H), 2.07 - 1.93 (m, 4H).

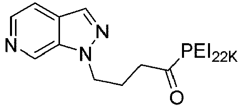

Compound **19** was prepared analogously to the general procedure, step 3 (Example 1). Yield= 100%; m= 84 mg; ¹H NMR (400 MHz, Deuterium Oxide) δ 9.67 - 8.10 (m, 4H), 4.23 - 3.06 (m, 16H), 2.86 - 1.90 (m, 4H).

### - Synthesis of compound 20 (Polymer Molecular weight = 22k, Heterocycle grafting = 22%)

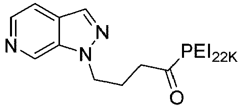

Compound 20 was prepared analogously to the general procedure, step 3 (Example 1). Yield= 89%; m= 68 mg; ¹H NMR (400 MHz, Deuterium Oxide) δ 8.63 - 6.72 (m, 4H), 4.57 - 2.78 (m, 20H), 2.75 - 1.47 (m, 4H).

### - Synthesis of compound 21 (Polymer Molecular weight = 22k, Heterocycle grafting = 21%)

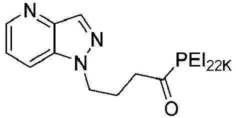

Compound **21** was prepared analogously to the general procedure, step 3 (Example 1). Yield= 97%; m= 73 mg; ¹H NMR (400 MHz, Deuterium Oxide) δ 9.12 - 7.56 (m, 4H), 4.58 - 4.36 (m, 2H), 4.09 - 3.01 (m, 19H), 2.90 - 1.77 (m, 4H).

### - Synthesis of compound 22 (Polymer Molecular weight = 22k, Heterocycle grafting = 21%)

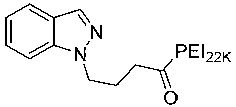

Compound **22** was prepared analogously to the general procedure, step 3 (Example 1). Yield= 52%; m= 39 mg; ¹H NMR (400 MHz, Deuterium Oxide) δ 8.21 - 6.38 (m, 5H), 4.47 - 1.37 (m, 25H).

### - Synthesis of compound 23 (Polymer Molecular weight = 22k, Heterocycle grafting = 26%)

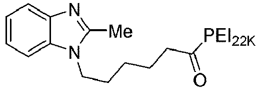

Compound **23** was prepared analogously to the general procedure, step 3 (Example 1). Yield= 97%; m= 169 mg; ¹H NMR (400 MHz, Deuterium Oxide) δ 7.74 - 7.19 (m, 4H), 4.41 - 3.00 (m, 13H), 2.83 - 2.52 (m, 3H), 2.48 - 1.99 (m, 2H), 1.95 - 1.00 (m, 6H).

### - Synthesis of compound 24 (Polymer Molecular weight = 8k, Heterocycle grafting = 20%)

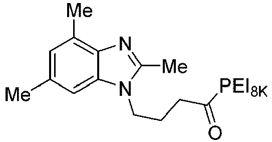

Compound **24** was prepared analogously to the general procedure, step 3 (Example 1). Yield= 70%; m= 22 mg; ¹H NMR (400 MHz, Deuterium Oxide) δ 7.80 - 6.15 (m, 2H), 4.50 - 3.21 (m,22H), 3.17 - 0.97 (m, 13H).

### - Synthesis of compound 25 (Polymer Molecular weight = 10k, Heterocycle grafting = 20%)

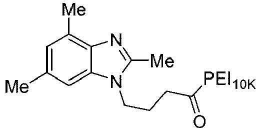

Compound **25** was prepared analogously to the general procedure, step 3 (Example 1). Yield= 73%; m= 23 mg; ¹H NMR (400 MHz, Deuterium Oxide) δ 7.98 - 6.15 (m, 2H), 4.62 - 3.06 (m, 22H), 2.99 - 1.39 (m, 13H).

### - Synthesis of compound 26 (Polymer Molecular weight = 15k, Heterocycle grafting = 17%)

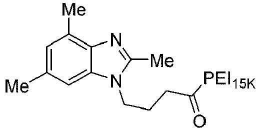

Compound **26** was prepared analogously to the general procedure, step 3 (Example 1). Yield= 84%; m= 25 mg; ¹H NMR (400 MHz, Deuterium Oxide) δ 7.64 - 6.35 (m, 2H), 4.57 - 3.13 (m, 25H), 3.13 - 1.50 (m, 13H).

### - Synthesis of compound 27 (Polymer Molecular weight = 30k, Heterocycle grafting = 18%)

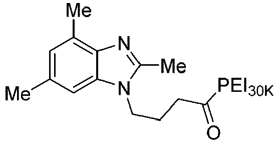

Compound **27** was prepared analogously to the general procedure, step 3 (Example 1). Yield= 68%; m= 21 mg; ¹H NMR (400 MHz, Deuterium Oxide) δ 7.74 - 6.35 (m, 2H), 4.50 - 3.01 (m, 24H), 2.97 - 1.41 (m, 13H).

### - Synthesis of compound 28 (Polymer Molecular weight = 25k, Heterocycle grafting = 22%)

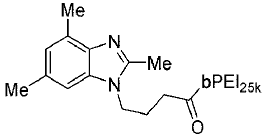

Compound **28** was prepared analogously to the general procedure, step 3 using branched polyethyleneimine (bPEI, 25K, Sigma-Aldrich). Yield= 94%; m= 282 mg;¹H NMR (400 MHz, Deuterium Oxide) δ 7.42 - 6.52 (m, 2H), 4.45 - 1.51 (m, 33H).

### - Synthesis of compound 29 (Polymer Molecular weight = 10k, Heterocycle grafting = 29%)

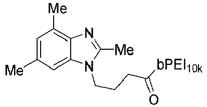

Compound **29** was prepared analogously to the general procedure, step 3 using branched polyethyleneimine (bPEI, 10K, Alfa Aesar). Yield= 99%; m= 351 mg; ¹H NMR (400 MHz, Deuterium Oxide) δ 7.37 - 6.50 (m, 2H), 4.57 - 1.44 (m, 29H).

### - Synthesis of compound 30 (Polymer Molecular weight = 22k, Heterocycle grafting = 35%)

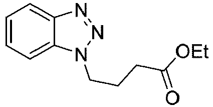

Intermediate **30a** was prepared analogously to the general procedure, step 1 (Example 1). Yield= 17%; m= 1.00 g; ¹H NMR (400 MHz, Chloroform-*d*) δ 7.95 (d, *J* = 8.4 Hz, 1H), 7.45 (d, *J* = 8.3 Hz, 1H), 7.38 (t, *J* = 7.6 Hz, 1H), 7.30 - 7.22 (m, 1H), 4.68 - 4.57 (m, 2H), 4.01 (qd, *J* = 7.1, 1.6 Hz, 2H), 2.30 - 2.16 (m, 4H), 1.13 (td, *J* = 7.1, 1.6 Hz, 3H).

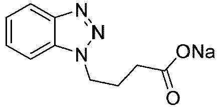

Intermediate **30b** was prepared analogously to the general procedure, step 2 (Example 1). Yield= 85%; m= 830 mg; ¹H NMR (400 MHz, Methanol-*d*₄) δ 8.03 - 7.96 (m, 1H), 7.82 (d, *J =* 8.5 Hz, 1H), 7.58 (t, *J* = 7.3 Hz, 1H), 7.45 (t, *J* = 8.0 Hz, 1H), 4.80 (dt, *J* = 7.0, 4.3 Hz, 2H), 2.37 (t, *J* = 7.0 Hz, 2H), 2.30 (q, *J* = 7.0 Hz, 2H).

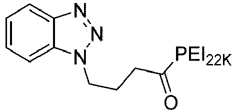

Compound **30** was prepared analogously to the general procedure, step 3 (Example 1). Yield= 100%; m= 189 mg; ¹H NMR (400 MHz, Deuterium Oxide) δ 7.41-6.55 (m, 4H), 4.58 - 3.02 (m, 14H), 2.90-1.31 (m, 3H).

### - Synthesis of compound 31 (Polymer Molecular weight = 22k, Heterocycle grafting = 35%)

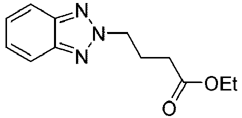

Intermediate **31a** was prepared analogously to the general procedure, step 1 (Example 1). Yield= 34%; m= 2.00 g; ¹H NMR (400 MHz, Chloroform-d) δ 7.73 (ddt, *J* = 7.4, 4.1, 2.2 Hz, 2H), 7.25 (ddt, *J* = 9.4, 4.0, 2.2 Hz, 2H), 4.68 (dd, *J* = 7.3, 5.5 Hz, 2H), 3.99 (ddd, *J* = 9.1, 7.2, 6.0 Hz, 2H), 2.40 -2.12 (m, 4H), 1.11 (tt, *J* = 7.3, 1.3 Hz, 3H).

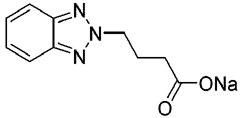

Intermediate **31b** was prepared analogously to the general procedure, step 2 (Example 1). Yield= 53%; m= 1.00 g; ¹H NMR (400 MHz, Methanol-*d*₄) δ 7.86 (dd, *J* = 6.9, 3.4 Hz, 2H), 7.42 (dd, *J* = 6.9, 3.4 Hz, 2H), 4.83 (d, *J* = 13.0 Hz, 2H), 2.37 (d, *J* = 4.3 Hz, 4H).

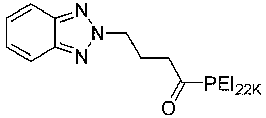

Compound **31** was prepared analogously to the general procedure, step 3 (Example 1). Yield= 100%; m= 166 mg; ¹H NMR (400 MHz, Deuterium Oxide) δ 7.83-6.50 (m, 4H), 4.61-3.90 (m, 2H), 3.88-2.51 (m, 11H), 2.49-1.35 (m, 4H).

### - Synthesis of compound 32 (Polymer Molecular weight = 22k, Heterocycle grafting = 22%)

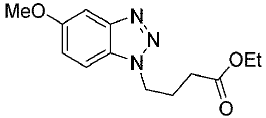

Intermediate **32a** was prepared analogously to the general procedure, step 1 (Example 1). Yield= 23%; m= 1.69 g; ¹H NMR (400 MHz, Chloroform-*d*) δ 7.78 (dd, *J* = 9.1, 1.9 Hz, 1H), 7.34 (dd, *J* = 9.1, 1.9 Hz, 0H), 7.26 (d, *J* = 2.3 Hz, 0H), 7.05 (dd, *J* = 9.0, 2.2 Hz, 0H), 6.89 (dd, *J* = 9.1, 2.2 Hz, 1H), 6.73 (d, *J* = 2.2 Hz, 1H), 4.55 (dtd, *J* = 13.6, 6.7, 1.9 Hz, 2H), 4.01 (q, *J* = 7.1 Hz, 2H), 3.78 (dd, *J* = 6.6, 1.8 Hz, 3H), 3.04 (s, 0H), 2.22 (ddd, *J* = 19.8, 7.7, 4.2 Hz, 4H), 1.13 (td, *J* = 7.1, 1.8 Hz, 3H).

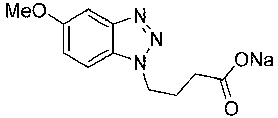

Intermediate **32b** was prepared analogously to the general procedure, step 2 (Example 1). Yield= 70%; m= 1.16 g; ¹H NMR (400 MHz, Methanol-*d*₄) δ 7.81 (dd, *J* = 9.1, 2.7 Hz, 1H), 7.67 (dd, *J* = 8.9, 2.7 Hz, 0H), 7.33 (s, 0H), 7.24 - 7.15 (m, 1H), 7.05 (dd, *J* = 9.1, 2.7 Hz, 1H), 4.73 (qd, *J* = 7.1, 2.3 Hz, 2H), 3.91 (dd, *J* = 13.2, 2.8 Hz, 3H), 3.33 (d, *J* = 3.2 Hz, 0H), 2.35 (t, *J* = 6.7 Hz, 2H), 2.26 (t, *J* = 7.3 Hz, 2H).

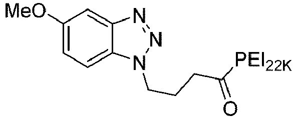

Compound **32** was prepared analogously to the general procedure, step 3 (Example 1). Yield= 96%; m= 153 mg; ¹H NMR (400 MHz, Deuterium Oxide) δ 7.99-6.22 (m, 3H), 4.49-4.03 (m, 2H), 3.96-2.80 (m, 21H), 2.63-1.55 (m, 4H).

### - Synthesis of compound 33 (Polymer Molecular weight = 22k, Heterocycle grafting = 18%)

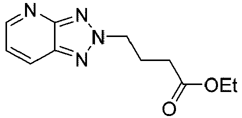

Intermediate **33a** was prepared analogously to the general procedure, step 1 (Example 1). Yield= 18%; m= 254 mg; ¹H NMR (400 MHz, Chloroform-d) δ 8.53 (dd, *J* = 4.4, 1.5 Hz, 1H), 7.82 (dd, *J* = 8.4, 1.5 Hz, 1H), 7.24 (dd, *J* = 8.4, 4.4 Hz, 1H), 4.54 (t, *J* = 6.8 Hz, 2H), 3.89 (q, *J* = 7.1 Hz, 2H), 2.20 - 2.05 (m, 4H), 1.01 (t, *J* = 7.1 Hz, 3H).

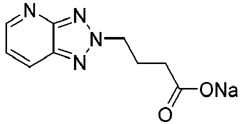

Intermediate **33b** was prepared analogously to the general procedure, step 2 (Example 1). Yield= 98%; m= 242 mg; ¹H NMR (400 MHz, Deuterium Oxide) δ 8.59 (dq, *J=* 4.5, 1.5 Hz, 1H), 8.21 (dt, *J* = 8.5, 1.6 Hz, 1H), 7.52 (ddt, *J* = 8.5, 4.5, 1.4 Hz, 1H), 4.70 - 4.62 (m, 2H), 2.21 - 2.04 (m, 4H).

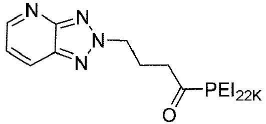

Compound **33** was prepared analogously to the general procedure, step 3 (Example 1). Yield= 75%; m= 54 mg; ¹H NMR (400 MHz, Deuterium Oxide) δ 9.06 - 6.76 (m, 3H), 4.16 - 2.96 (m, 20H), 2.93 - 1.52 (m, 4H).

### - Synthesis of compound 34 (Polymer Molecular weight = 22k, Heterocycle grafting = 17%)

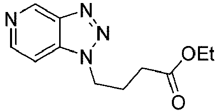

Intermediate **34a** was prepared analogously to the general procedure, step 1 (Example 1). Yield= 52%; m= 600 mg; ¹H NMR (400 MHz, Chloroform-d) δ 9.51 - 9.16 (m, 1H), 8.60 - 8.50 (m, 1H), 7.96 - 7.52 (m, 1H), 4.90 - 4.71 (m, 2H), 4.10 (dq, *J* = 8.6, 7.1 Hz, 2H), 2.43 - 2.30 (m, 4H), 1.23 (t, *J* = 7.2 Hz, 3H).

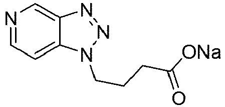

Intermediate **34b** was prepared analogously to the general procedure, step 2 (Example 1). Yield= 97%; m= 550 mg; ¹H NMR (400 MHz, Deuterium Oxide) δ 9.27 - 9.11 (m, 1H), 8.43 - 8.27 (m, 1H), 7.95 - 7.69 (m, 1H), 4.84 - 4.63 (m, 2H), 2.28 - 2.05 (m, 4H).

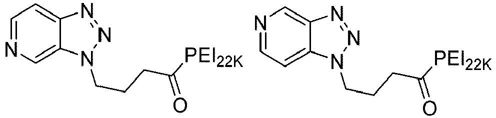

Compound **34** was prepared analogously to the general procedure, step 3 (Example 1). Yield= 95%; m= 68 mg; ¹H NMR (400 MHz, Deuterium Oxide) δ 10.08 - 8.17 (m, 3H), 4.21 - 2.84 (m, 25H), 2.83 - 1.64 (m, 4H).

### Example 3. Thermal inactivation of viral vectors.

Heat inactivation of viral vectors is often performed around 70 °C in the presence of 0.05-0.1% SDS (Sommer JM, et al. Molecular therapy: the journal of the American Society of Gene Therapy 2003, 7(1), 122-128*;* Gargi Maheshwari, et al. Journal of Virological Methods 2004, 118(2) 141-146*;* Fabian Krieselet al. Journal of Virological Methods 2020, 276, 113768*).* It usually takes one hour to perform these inactivation protocols. However, to avoid the addition of chemical and to reduce heating time, the inventors studied the thermal inaction at 120 °C for 30 minutes.

Therefore, the stability of **compound 05** at 120 °C over 24 hours was evaluated. An overlay of the different chromatograms at different timepoints is represented in **Figure 3****.** No degradation was observed over 4 hours. After 6 hours, peaks were detected around **compound 05** of interest meaning a slight degradation. Based on this stability study, the possible residual reagent in the sample is not affected by the autoclave treatment during 30 minutes.

### Example 4. Optimization of acidic hydrolysis.

The inventors studied 3 different parameters to define the best conditions where **compound 05** will not be degraded:
- Type of acids,
- Temperature,
- Heating time.

Acidic hydrolysis with 20 ppm of **compound 05** was performed with HCl, H₂SO₄, HNO₃, which are 3 acids known to degrade proteins or nucleic acid.

To a solution of **compound 05** (20 ppm) in distillated water was added respectively chlorhydric, sulfuric or nitric acid. The 3 mixtures were heated at 100°C overnight. For each acid, the following methodology was followed:
- Accuracy on reference substance **compound 05** in triplicate. This test consists in hydrolyzing the reference substance at 20ppm with the different acid and perform the treatment on a centrifugal filter. The acidic hydrolysis with HCl was performed over 46 hours (15 hours for the other acids).
- Recovery on the virus spiked at 20 ppm with **compound 05.** This parameter was determined on 3 injections.

The different results are summarized in **Table 3** and demonstrate that any condition is able to avoid degradation of **compound 05.** However, chlorhydric acid demonstrates the best reproducibility compared to sulfuric or nitric acids.

**Table 3. Recovery results with different acids used for the acidic hydrolysis.**

| | Virus spiked at 20 ppm with **compound 05** | | | | |
|---|---|---|---|---|---|
| Acid type | Recovery - Injection 1 | Recovery - Injection 2 | Recovery - Injection 3 | Average | Standard deviation |
| HCl - after 15 hours | 49.5 | 40.0 | 54.0 | 47.8 | 15.0 |
| HCl - after 46 hours | 18.8 | 18.8 | 20.0 | 19.2 | 3.5 |
| H₂SO₄ | 42.1 | 79.9 | 75.6 | 65.8 | 31.4 |
| HNO₃ | 3.5 | 6.6 | 34.4 | 14.8 | 115 |

Concentration of HCl and heating time are critical for the acidic hydrolysis. Thus the inventors evaluated these 2 criteria:
- Concentration of HCl: 10%, 1%, 0.1%,
- Heating time at 60°C, 80°C, 100°C: t0, +1h, +2h, +3h, +4h, +5h, +6, +24h.

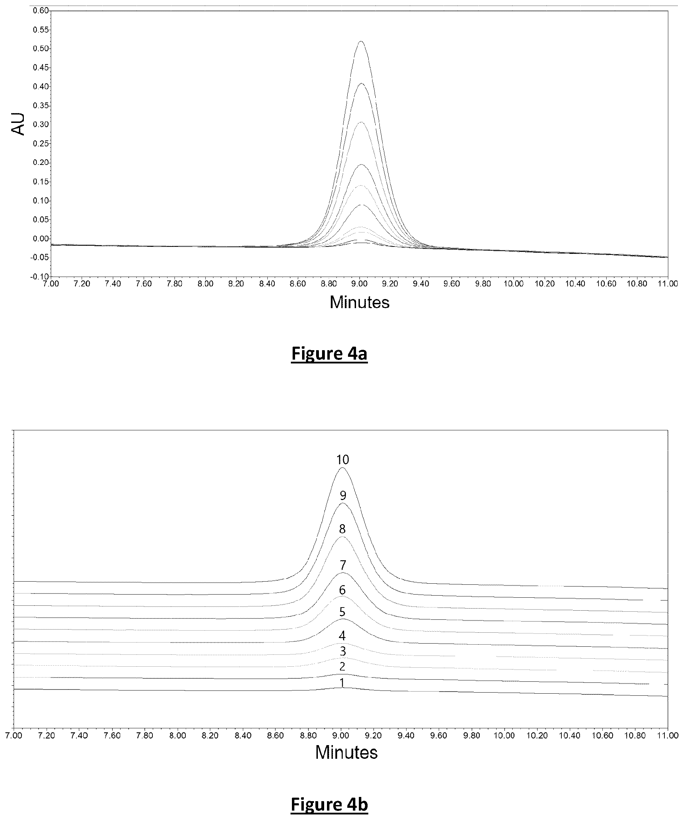

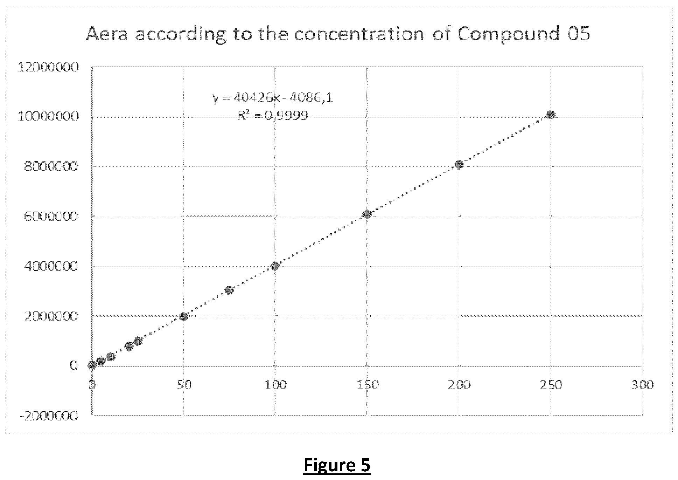

Finally, the optimized conditions were fixed to an acidic hydrolysis in a chlorhydric solution 0.1% at 100°C for 2 hours. Linearity in **Figure 4a, Figure 4b** and **Figure 5** shows a LOD = 1.97 and LOQ = 6.55.

**Figure 4b** shows an overlay of the **Figure 4a****.** 1 represents the compound 5, 5ppm at 110°C; 2 represents the compound 5, 10ppm at 110°C; 3 represents the compound 5, 20ppm at 110°C; 4 represnets the compound 5, 25ppm at 110°C; 5 represents the compound 5, 50ppm at 110°C; 6 represents the compound 6, 75ppm at 110°C; 7 represents the compound 5, 100ppm at 110°C; 8 represents the compound 5, 150 ppm at 110°C.

### Example 5. Qualitative and quantitative analysis of the residual product.

The HPLC method is described in **Table 4** below. This analytical method was developed during the whole development of the invention because of two main issues:
- Presence of carry-over which could impact the quantification,
- The symmetry of the different peaks which was not optimal considering a potential validation of the method.

Different "in-house" tests were performed by the inventors in order to tackle the carry-over issue by varying different parameters such as the column type (grafted C4 vs C18, AX vs Peptide), the mobile phases composition, gradient slope and flow rate. After those different screenings, a new analytical method was put in place and is described in **Table 4** below.

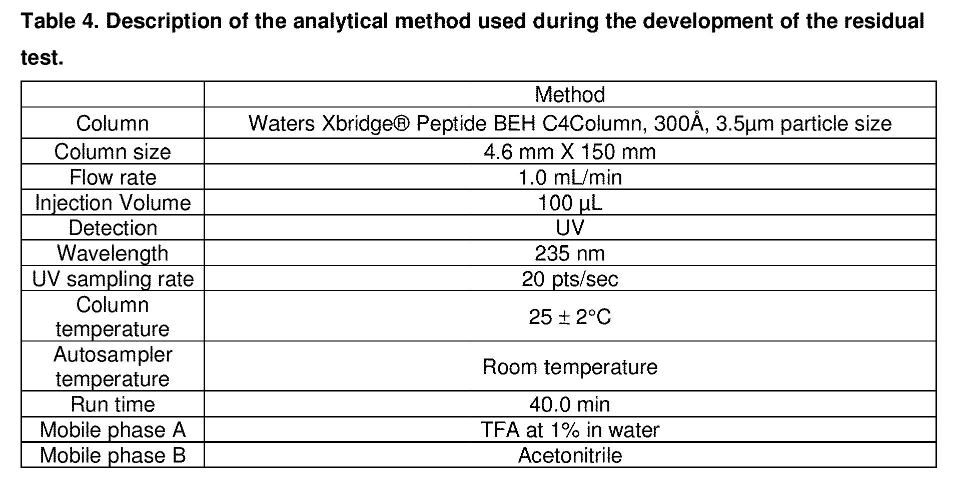

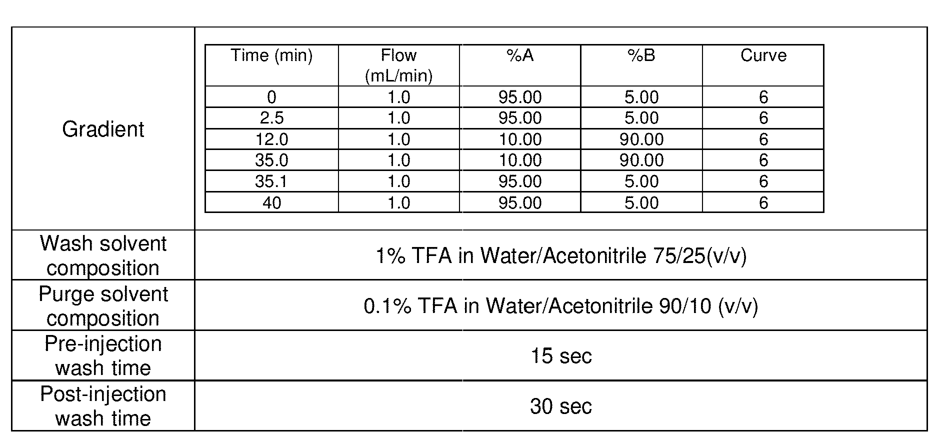

### Example 6. Experimental section.

The inventors demonstrated the method efficiency on a variety of transfection reagents (for example as described in WO2021/023796; WO2021/023798) which showed strong activity in transfection, in particular for viral vector's production.

The inventors studied the stability of all the transfection reagentscompounds 01 to 34 under the acidic hydrolysis described previously (**Table 5**)**.** Most of the molecules were fully stable under the acidic conditions. Moreover, based on the study of both limit of detection (LOD) and limit of quantification (LOQ), the analytical method was applicable to a large variety of PEI-based polymers.

**Table 5. Examples of polymers used in the present invention (n.d.= not detected, experiments: A transfection reagent (20 ppm) in a solution of HCl (2 mL, 0.1%) was heated at 110°C for 2 hours. After cooling, the experiment was directly analyzed by HPLC.)**

| **Compounds** | **Structures** | **Polymer** | **Graf -ting (%)** | **LOD (ppm)** | **LOO (ppm)** | **Degree of hydrolysis (%)** |
|---|---|---|---|---|---|---|
| 01 | 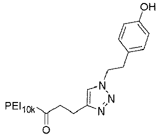 | IPEI₁₀ₖ | 16 | 7.4 | 24,68 | n.d. |
| 02 | 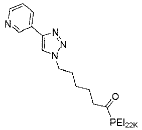 | IPEI₂₂ₖ | 18 | 0.73 | 2.42 | n.d. |
| 03 | 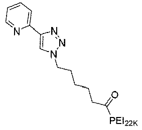 | IPEI₂₂ₖ | 18 | 0.76 | 2.52 | n.d. |
| 04 | 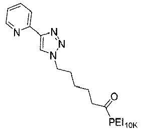 | IPEI₁₀ₖ | 22 | 0.42 | 1.40 | n.d. |
| 05 | 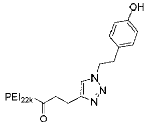 | IPEI₂₂ₖ | 22 | 2,52 | 8,41 | n.d. |
| 06 | 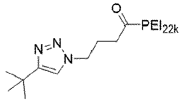 | IPEI₂₂ₖ | 16 | 3.18 | 10.61 | n.d. |
| 07 | 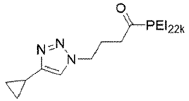 | IPEI₂₂ₖ | 19 | 0.94 | 3.14 | n.d. |
| 08 | 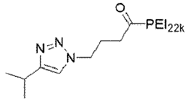 | IPEI₂₂ₖ | 19 | 3.91 | 13.02 | n.d. |
| 09 | 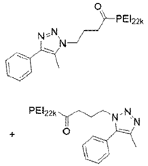 | IPEI₂₂ₖ | 22 | 3.49 | 11.62 | n.d. |
| 10 | 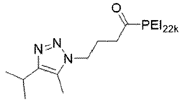 | IPEI₂₂ₖ | 21 | 2.14 | 7.14 | n.d. |
| 11 | 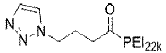 | IPEI₂₂ₖ | 25 | 2.96 | 9.86 | n.d. |
| 12 | 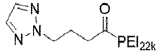 | IPEI₂₂ₖ | 25 | 0.51 | 1.69 | n.d. |
| 13 | 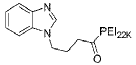 | IPEI₂₂ₖ | 10 | 0.73 | 2.42 | n.d. |
| 14 | 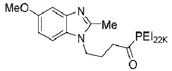 | IPEI₂₂ₖ | 24 | 0.65 | 2.16 | n.d. |
| 15 | 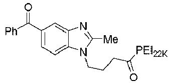 | IPEI₂₂ₖ | 27 | 1.92 | 6.39 | n.d. |
| 16 | 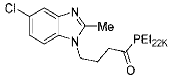 | IPEI₂₂ₖ | 30 | 1.49 | 4.97 | n.d. |
| 17 | 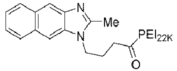 | IPEI₂₂ₖ | 26 | 0.17 | 0.58 | n.d. |
| 18 | 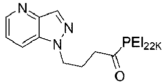 | IPEI₂₂ₖ | 47 | 0.42 | 1.39 | n.d. |
| 19 | 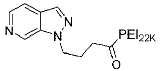 | IPEI₂₂ₖ | 25 | 0.24 | 0.81 | n.d. |
| 20 | 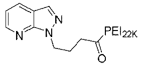 | IPEI₂₂ₖ | 22 | 0.94 | 3.12 | n.d. |
| 21 | 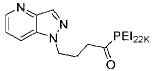 | IPEI₂₂ₖ | 21 | 0.26 | 0.85 | n.d. |
| 22 | 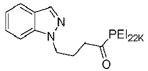 | IPEI₂₂ₖ | 21 | 5.83 | 19.45 | n.d. |
| 23 | 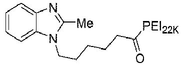 | IPEI₂₂ₖ | 26 | 0.31 | 1.04 | n.d. |
| 24 | 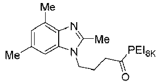 | IPEI₂₂ₖ | 11 | 2.93 | 9.76 | n.d. |
| 25 | 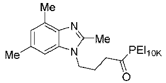 | IPEI₁₀ₖ | 20 | 1.90 | 6.33 | n.d. |
| 26 | 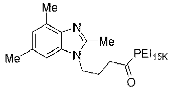 | IPEI₁₅ₖ | 17 | 2.53 | 8.43 | n.d. |
| 27 | 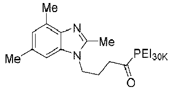 | IPEI₃₀ₖ | 18 | 1.87 | 6.23 | n.d. |
| 28 | 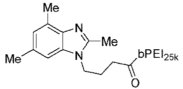 | bPEI₂₅ₖ | 22 | 9,32 | 31,07 | 4 |
| 29 | 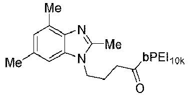 | bPEI₁₀ₖ | 29 | 8,78 | 29,25 | 9 |
| 30 | 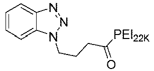 | IPEI₂₂ₖ | 35 | 4.11 | 13.7 | n.d. |
| 31 | 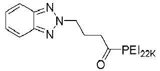 | IPEI₂₂ₖ | 35 | 7,72 | 25,72 | n.d. |
| 32 | 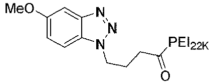 | IPEI₂₂ₖ | 22 | 10,58 | 35,26 | n.d. |
| 33 | 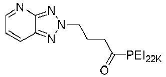 | IPEI₂₂ₖ | 18 | 0.8 | 2.67 | 23 |
| 34 | 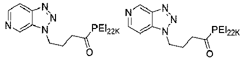 | IPEI₂₂ₖ | 17 | 0,84 | 2,79 | n.d. |

Then the inventors applied the whole residual test described above on a selection of molecules listed in **Table 6** below.

**Table 6. Residual test. n.d.= not detected; Procedure for spike (adding a known quantity of transfection agent; Araujo, Journal of Chromatography 6, 2009, 877(23), 2224-2234) before hydrolysis: to a solution of viral vector (AAV, 200 µL) was added a solution of HCl (0.37%, 108 µL), a volume of pure water (92 µL) and a solution of transfection reagent (conc: 100 ppm, 80 µL).**

| **Compounds** | **Structures** | **LOD (ppm)** | **LOQ (ppm)** | **Recovery (%)** | **Degree of hydrolysis** |
|---|---|---|---|---|---|
| 27 | 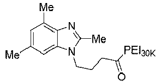 | 1.87 | 6.23 | Spike before hydrolysis: **73** | n.d. |
| | | | | Spike after hydrolysis: **80** | |
| 04 | 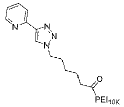 | 0.42 | 1.40 | Spike before hydrolysis: **89** | n.d. |
| | | | | Spike after hydrolysis: **89** | |
| 07 | 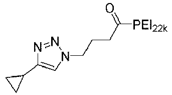 | 0.94 | 3.14 | Spike before hydrolysis: **59** | n.d. |
| | | | | Spike after hydrolysis: **93** | |
| 10 | 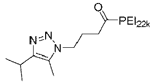 | 2.14 | 7.14 | Spike before hydrolysis: **78** | n.d. |
| | | | | Spike after hydrolysis: **85** | |
| 14 | 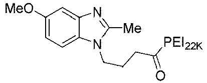 | 0.65 | 2.16 | Spike before hydrolysis: **89** | n.d. |
| | | | | Spike after hydrolysis: **82** | |
| 17 | 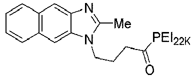 | 0.17 | 0.58 | Spike before hydrolysis: **72** | n.d. |
| | | | | Spike after hydrolysis: **89** | |
| 26 | 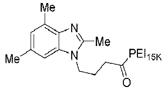 | 2,56 | 8,43 | Spike before hydrolysis: **82** | n.d. |
| | | | | Spike after hydrolysis: **85** | |
| 22 | 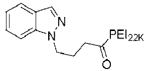 | 5.83 | 19.45 | Spike before hydrolysis: **75** | n.d. |
| | | | | Spike after hydrolysis: **82** | |
| 19 | 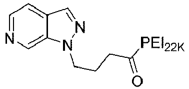 | 0.24 | 0.81 | Spike before hydrolysis: **73** | n.d. |
| | | | | Spike after hydrolysis: **76** | |
| 05 | 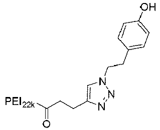 | 2,52 | 8,41 | Spike before hydrolysis: **84** | n.d. |
| | | | | Spike after hydrolysis: **68** | |

The tube was sealed then heated at 100°C for 2 hours. After cooling to room temperature, the mixture was diluted in 3.5 mL pure water then filtered by centrifugation using a centrifugal filter with a minimal cut-off ok 1kDa for 30 minutes at 5.000 g to afford a solution between 250-300µL. The solution was completed to 4mL and filtered at 5.000 g for 60 minutes. The same procedure was repeated 2 more times. The final retentate was diluted with pure water to give a solution of 500 µL and analyzed by HPLC.

Procedure for spike after hydrolysis: to a solution of viral vector (AAV, 200 µL) was added a solution of HCl (0.37%, 108 µL), a volume of pure water (92 µL) and a solution of transfection reagent (cone: 100 ppm, 80µL). The tube was sealed then heated at 100°C for 2 hours. After cooling, a solution of transfection reagent (conc: 100 ppm, 80 µL) was added before the filtration process.
Examples of buffers used with **compound 17** are disclosed in **Table 7.**

**Table 7. Examples of buffer with compound 17.**

| **buffer** | **Recovery (%)** | **Degree of hydrolysis** |
|---|---|---|
| **HEPES** | Spike before hydrolysis: **26** | **n.d.** |
| | Spike after hydrolysis: **51** | |
| **D-PBS** (Na₂HPO₄ 10mM + KH₂PO₄ 1,8mM pH=7.4 + KCl 27mM + NaCl 137mM) | Spike before hydrolysis: **66** | **n.d.** |
| | Spike after hydrolysis: **84** | |
| **NaCl** (150 mM) | Spike before hydrolysis: **76** | **n.d.** |
| | Spike after hydrolysis: **86** | |
| **Sodium citrate** (0.1M, pH3) | Spike before hydrolysis: **60** | **n.d.** |
| | Spike after hydrolysis: **65** | |
| **BalanCD HEK** | Spike before hydrolysis: **60** | **n.d.** |
| | Spike after hydrolysis: **77** | |
| **Freestyle 293** | Spike before hydrolysis: **85** | **n.d.** |
| | Spike after hydrolysis: **84** | |
| **Freestyle F17** | Spike before hydrolysis: **83** | **n.d.** |
| | Spike after hydrolysis: **88** | |
| **DMEM** (Low Glucose 1 g/L) | Spike before hydrolysis: **64** | **n.d.** |
| | Spike after hydrolysis: **72** | |
| **DMEM** (High Glucose 4.5 g/L) | Spike before hydrolysis: **55** | **n.d.** |
| | Spike after hydrolysis: **59** | |
| **OPTIMEM** | Spike before hydrolysis: **50** | **n.d.** |
| | Spike after hydrolysis: **74** | |
| **D-PBS (+ sucrose 1M)** | Spike before hydrolysis: **43** | **n.d.** |
| | Spike after hydrolysis: **78** | |
| **BSS** (NaCl 109,5mM + KCl 10,1mM + CaCl₂ 3,3mM + MgCl₂ 1,4mM + C₂H₃NaO₂ 28mM + Na₃C₆H₅O₇ 5,8 pH7,4 a 0,02% Tween) | Spike before hydrolysis: **62** | **n.d.** |
| | Spike after hydrolysis: **73** | |
| **D-PBS at 10% Glycerol** | Spike before hydrolysis: **71** | **n.d.** |
| | Spike after hvdrolysis: **79** | |
| **Buffer 1:** D-PBS at 6% Sorbitol at 0,05% Poloxamer 10% | Spike before hydrolysis: **87** | **n.d.** |
| | Spike after hydrolysis: **94** | |
| **Buffer 2:** Glycine 50mM + Tris 10Mm + NaCl 1000mM + 2% Saccharose + 0,2% Pluronic in purified water | Spike before hydrolysis: **0** | **n.d.** |
| | Spike after hydrolysis: **83** | |
| | Spike before hydrolysis: **14** | **n.d.** |
| **Buffer 3:** 5% Tween 80 + HEPES 50mM + NaCl 400mM + MgCl2 20mM + 1% Saccharose in Freestyle F17 | Spike after hydrolysis: **4** | |
| **Buffer 4:** 1% Tween 80 + HEPES 50mM + NaCl 400mM + MgCl2 20mM + 1% saccharose in Freestyle F17 | Spike before hydrolysis: **21** | **n.d.** |
| | Spike after hydrolysis: **10** | |
| **Buffer 5:** Tris 20mM + NaCl 400mM + 1% Saccharose + 5% Tween 80 in purified water | Spike before hydrolysis: **12** | **n.d.** |
| | Spike after hydrolysis: **6** | |
| **Buffer 6:** HEPES 20mM + MES 20mM + Sodium Acetate 20mM + NaCl 150mM + CaCl2 5mM in purified water | Spike before hydrolysis: **71** | **n.d.** |
| | Spike after hydrolysis: **77** | |

## Claims

1. A method for performing an acidic hydrolysis of a liquid mixture comprising a biological matrix and a polyethyleneimine (PEI)-based transfection reagent,
wherein the biological matrix comprises a recombinant virus or virus-like particles produced using the PEI-based transfection reagent,
wherein the method comprises the step of incubating the liquid mixture comprising the biological matrix in an aqueous solution comprising from 0.1% to 10% (v/v) hydrochloric acid (HCl) at a temperature ranging from 60°C to 110°C for a time period ranging from 2 hours to 24 hours, preferably an aqueous solution comprising 0.1% (v/v) HCl at a temperature of 110°C for 2 hours or in an aqueous solution comprising 1% (v/v) HCl at a temperature ranging from 60°C to 80°C for 2 hours,
wherein said acidic hydrolysis does not degrade the PEI-based transfection reagent, and wherein the PEI-based transfection reagent is of general formula (I) or an acceptable salt thereof: 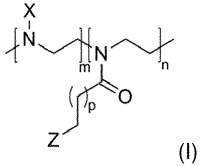
wherein:
- m represents an integer between 27 to 1200 and n represents an integer between 3 to 600, with the proviso that n is lower than m and the sum of m+n ranges from 30 to 1200,
- X represents H or a group of formula: 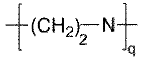 in which q represents an integer between 10 and 800,
- p represents an integer between 1 and 4,
- Z represents a group of formula: 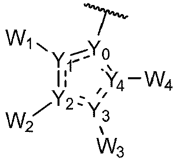
- Y₀, Y₁, Y₂, Y₃ and Y₄, which may be identical or different, represent C or N, with the proviso that at least two, but no more than three, of Y₀, Y₁, Y₂, Y₃ and Y₄, are N,
- W₁, W₂, W₃ and W₄, which may be identical or different, represent H, a linear or branched, saturated or unsaturated C₁-C₁₈ alkyl, C₆-C₁₈ aryl, a linear or branched, saturated or unsaturated C₆-C₁₈ aryl-C₁-C₁₈ alkyl, C₅-C₁₀ heteroaryl, a linear or branched, saturated or unsaturated C₂-C₁₈ heteroalkyl, an amine, a linear or branched, saturated or unsaturated C₁-C₁₈ alkylamine, a C₁₋C₁₂ alkoxy; or (i) W₁ and W₂ or (ii) W₂ and W₃ or (iii) W₃ and W₄ together form a fused, optionally substituted six-membered aryl; or a fused, optionally substituted six-membered heteroaryl containing no more than 1 N atom,
with the proviso that at least one, but no more than two, of W₁, W₂, W₃ and W₄ is, or are, absent.

2. The method according to claim 1, wherein W₁, W₂, W₃ and W₄, which may be identical or different, represent H, methyl, cyclopropyl, isopropyl, tert-butyl, phenyl, benzyl, 2-pyridine, 3-pyridine, or 4-hydroxyphenethyl; or wherein (i) W₁ and W₂ or (ii) W₂ and W₃ or (iii) W₃ and W₄ together form a fused phenyl; a fused phenyl substituted by a methyl group, in particular two methyl groups, a methoxy group, a carboxyphenyl or Cl; a fused naphthalene; a fused 2-pyridine; or a fused 3-pyridine.

3. The method according to claim 1 or 2, wherein X represents H.

4. The method according to any one of claims 1 to 3, wherein the PEI-based transfection reagent of general formula (I) has a grafting ratio defined as (n/(m+n))*100, and wherein the grafting ratio is ranging from 1 to 50%, preferably from 5 to 30%, more preferably is 20%.

5. The method according to any one of claims 1 to 4, wherein the PEI polymer backbone has an average molecular weight (Mw) ranging from 1 kDa to 50 kDa, preferably from 5 kDa to 30 kDa or from 10 kDa to 25 kDa, more preferably the PEI polymer backbone has an average molecular weight (Mw) of 8, 10, 15, 22, 25 or 30 kDa, preferably of 22 kDa.

6. The method according to any one of claims 1 to 5, wherein the PEI-based transfection reagent of general formula (I) is selected from the group consisting of the following compounds:

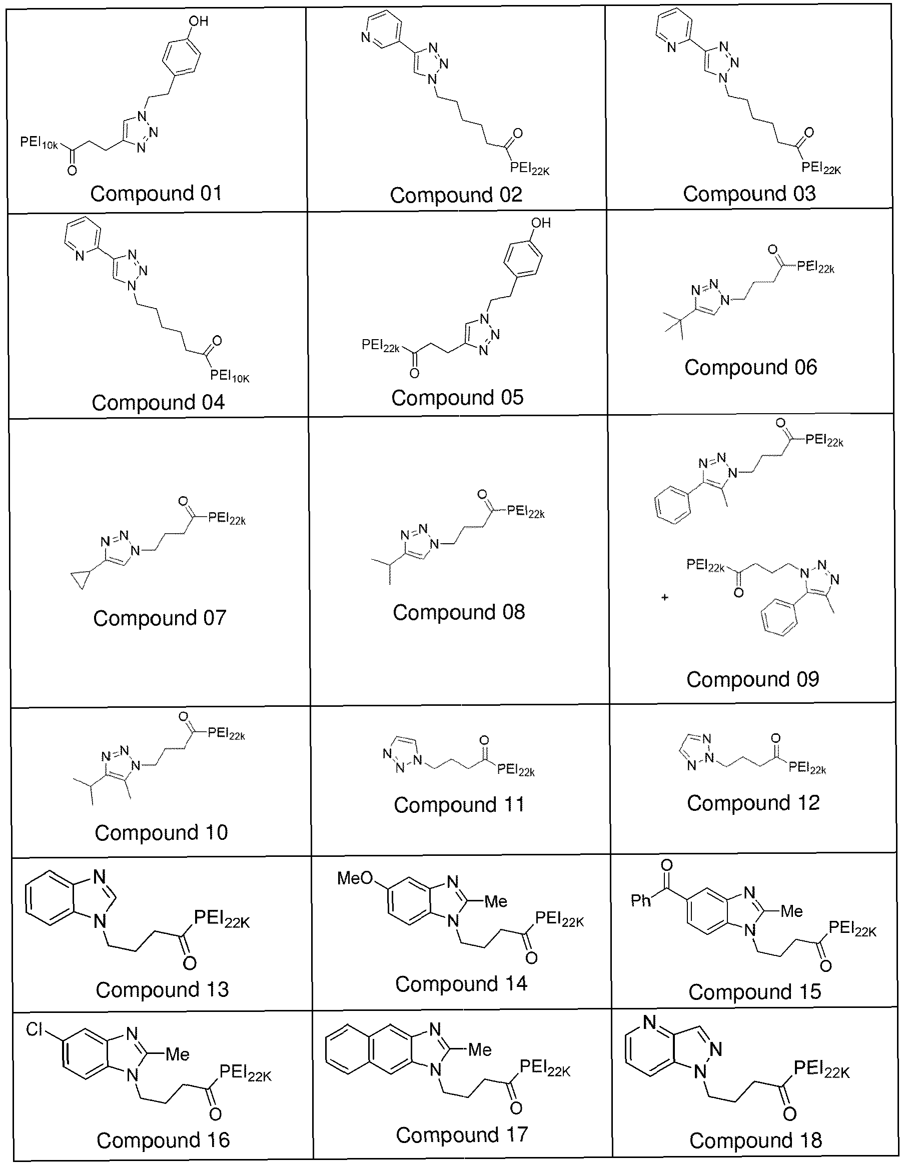

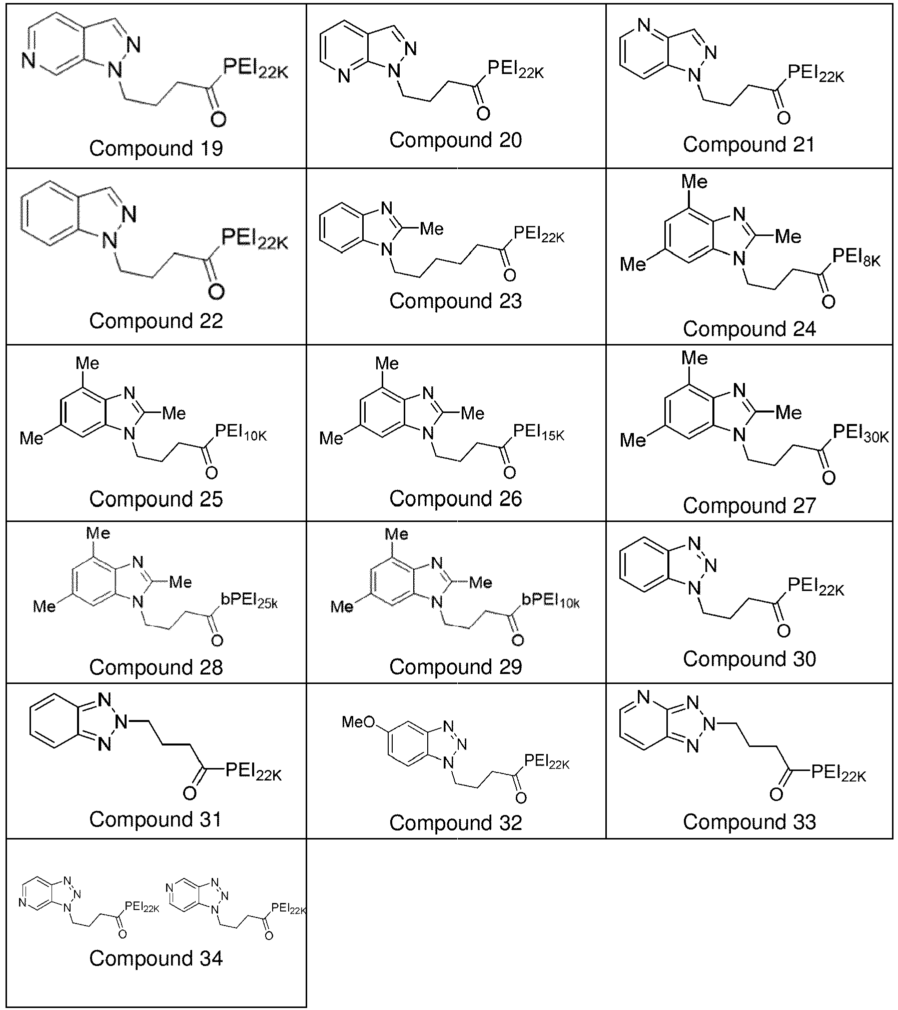

7. The method according to any one of claims 1 to 6, wherein the biological matrix is selected from the group consisting of a cell culture medium, in particular a culture medium of eukaryotic cells, in particular suspension cells or adherent cells, a buffer, a solution used during the manufacturing and purification process of recombinant viruses, and a final composition comprising the purified manufactured recombinant viruses in a final formulation comprising pharmaceutically acceptable buffer and excipients.

8. A method for purifying, detecting and/or quantifying a polyethyleneimine (PEI)-based transfection reagent of general formula (I) as defined in any one of claims 1 to 6, wherein the PEI-based transfection reagent of general formula (I) is comprised in a liquid mixture comprising a biological matrix,
wherein the biological matrix comprises a recombinant virus or virus-like particles produced using the PEI-based transfection reagent of general formula (I),
wherein the method comprises the steps of:
(a) performing an acidic hydrolysis of the liquid mixture according to the method of any one of claims 1 to 7,
(b) purifying the reaction mixture obtained in step (a) in order to obtain a purified PEI-based transfection reagent of general formula (I),
(c) detecting and/or quantifying the purified PEI-based transfection reagent of general formula (I) obtained in step (b).

9. The method according to claim 8, wherein step (c) is performed using High-performance liquid chromatography (HPLC) or Ultra high-performance liquid chromatography (UHPLC) analytical technique, preferably UHPLC.

10. The method according to claim 8 or 9, wherein the PEI-based transfection reagent of general formula (I) of step (c) is detected with a limit of detection (LOD) ranging from 1 ppm to 1000 ppm, and/or a limit of quantification (LOQ) ranging from 1 ppm to 1000 ppm.

11. The method according to any one of claims 1 to 10, wherein the PEI-based transfection reagent of general formula (I) is detectable in a biological matrix during the manufacturing process of the recombinant viruses, wherein the biological matrix is selected from the group consisting of a cell culture medium, in particular a culture medium of eukaryotic cells, in particular suspension cells or adherent cells, a buffer, a solution used during the manufacturing and purification process of recombinant viruses, and a final composition comprising purified viruses in a final formulation comprising pharmaceutically acceptable buffer and excipients.

12. The method according to any one of claims 1 to 11, wherein the recombinant virus is selected from the group consisting of an adeno-associated virus (AAV), a lentivirus (LV), an adenovirus, an oncolytic virus and a baculovirus, preferably is an adeno-associated virus (AAV) or a lentivirus (LV), more preferably is an adeno-associated virus (AAV).

13. The method according to any one of claims 6 to 12, wherein the PEI-based transfection reagent of general formula (I) is a compound selected from the group consisting of compounds 01, 02, 03, 04, 05, 06, 07, 08, 09, 10, 11, 12, 30, 31, 32, 33 and 34, and the recombinant virus is an adeno-associated virus (AAV).

## Patentansprüche

1. Verfahren zur sauren Hydrolyse einer flüssigen Mischung, die eine biologische Matrix und ein Polyethylenimin(PEI)-basiertes Transfektionsreagenz umfasst,
wobei die biologische Matrix ein rekombinantes Virus oder Viruspartikel umfasst, die unter Verwendung des PEI-basierten Transfektionsreagenzes hergestellt wurden,
wobei das Verfahren den Schritt des Inkubierens der die biologische Matrix umfassenden flüssigen Mischung in einer wässrigen Lösung mit 0,1 ö bis 10 ö (v/v) Salzsäure (HCl) bei einer Temperatur zwischen 60 °C und 110 °C für einen Zeitraum von 2 Stunden bis 24 Stunden umfasst, vorzugsweise in einer wässrigen Lösung mit 0,1 ö (v/v) HCl bei einer Temperatur von 110 °C für 2 Stunden, oder in einer wässrigen Lösung mit 1 ö (v/v) HCl bei einer Temperatur zwischen 60 °C und 80 °C für 2 Stunden,
wobei die genannte saure Hydrolyse das PEI-basierte Transfektionsreagenz nicht abbaut, und
das PEI-basierte Transfektionsreagenz die allgemeine Formel (I) oder ein akzeptables Salz davon ist: 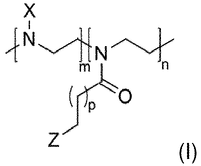
wobei:
- m eine ganze Zahl zwischen 27 und 1200 und n eine ganze Zahl zwischen 3 und 600 ist, mit der Maßgabe, dass n kleiner m ist und die Summe von m+n zwischen 30 und 1200 liegt,
- X für H oder eine Formelgruppe steht: 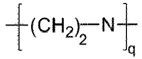
wobei q eine ganze Zahl zwischen 10 und 800 ist,
- p eine Zahl zwischen 1 und 4 ist,
- Z eine Formelgruppe ist: 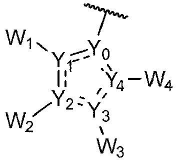
- Y₀, Y₁, Y₂, Y₃ und Y₄, die identisch oder verschieden sein können, für C oder N stehen, mit der Maßgabe, dass mindestens zwei, jedoch nicht mehr als drei der Y₀, Y₁, Y₂, Y₃ und Y₄ für N stehen,
- W₁, W₂, W₃ und W₄, die identisch oder verschieden sein können, für H, ein geradkettiges oder verzweigtes, gesättigtes oder ungesättigtes C₁-C₁₈-Alkyl, ein geradkettiges oder verzweigtes C₆-C₁₈-Aryl-C₁-C₁₈-Alkyl, ein C₅-C₁₀-Heteroaryl, ein geradkettiges oder verzweigtes, gesättigtes oder ungesättigtes C₂-C₁₈-Heteroalkyl, ein Amin, ein lineares oder verzweigtes, gesättigtes oder ungesättigtes C₁-C₁₈-Alkylamin, ein C₁-C₁₂-Alkoxy stehen; oder (i) W₁ und W₂ oder (ii) W₂ und W₃ oder (iii) W₃ und W₄ zusammen ein gegebenenfalls substituiertes kondensiertes sechsgliedriges Aryl oder ein gegebenenfalls substituiertes kondensiertes sechsgliedriges Heteroaryl mit nicht mehr als einem N-Atom bilden, mit der Maßgabe, dass mindestens eines, aber nicht mehr als zwei der W₁, W₂, W₃ und W₄ nicht vorhanden ist bzw. sind.

2. Verfahren nach Anspruch 1, in dem W₁, W₂, W₃ und W₄, die identisch oder verschieden sein können, für H, Methyl, Cyclopropyl, Isopropyl, tert-Butyl, Phenyl, Benzyl, 2-Pyridin, 3-Pyridin oder 4-Hydroxyphenyl stehen; oder in dem (i)W₁ und W₂ oder (ii) W₂ und W₃ oder (iii) W₃ und W₄ zusammen ein kondensiertes Phenyl bilden; ein kondensiertes Phenyl, das durch eine Methylgruppe, insbesondere zwei Methylgruppen, eine Methoxygruppe, ein Carboxyphenyl oder Cl substituiert ist; ein kondensiertes Naphthalin; ein kondensiertes 2-Pyridin; oder ein kondensiertes 3-Pyridin.

3. Verfahren nach Anspruch 1 oder Anspruch 2, in dem X für H steht.

4. Verfahren nach einem der Ansprüche 1 bis 3, in dem das PEI-basierte Transfektionsreagenz der allgemeinen Formel (I) einen Pfropfgrad aufweist, der als (n/(m+n))*100 definiert ist, und der Pfropfgrad zwischen 1 und 50 %, vorzugsweise zwischen 5 und 30 ö und besonders bevorzugt 20 ö liegt.

5. Verfahren nach einem der Ansprüche 1 bis 4, in dem das PEI-Polymergerüst ein mittleres Molekulargewicht (Mw) zwischen 1 kDa und 50 kDa, vorzugsweise zwischen 5 kDa und 30 kDa oder zwischen 10 kDa und 25 kDa aufweist bzw. das PEI-Polymergerüst insbesondere bevorzugt ein mittleres Molekulargewicht (Mw) von 8, 10, 15, 22, 25 oder 30 kDa, vorzugsweise von 22 kDa, aufweist.

6. Verfahren nach einem der Ansprüche 1 bis 5, in dem das PEI-basierte Transfektionsreagenz der allgemeinen Formel (I) aus der Gruppe ausgewählt ist, die aus folgenden Verbindungen besteht:

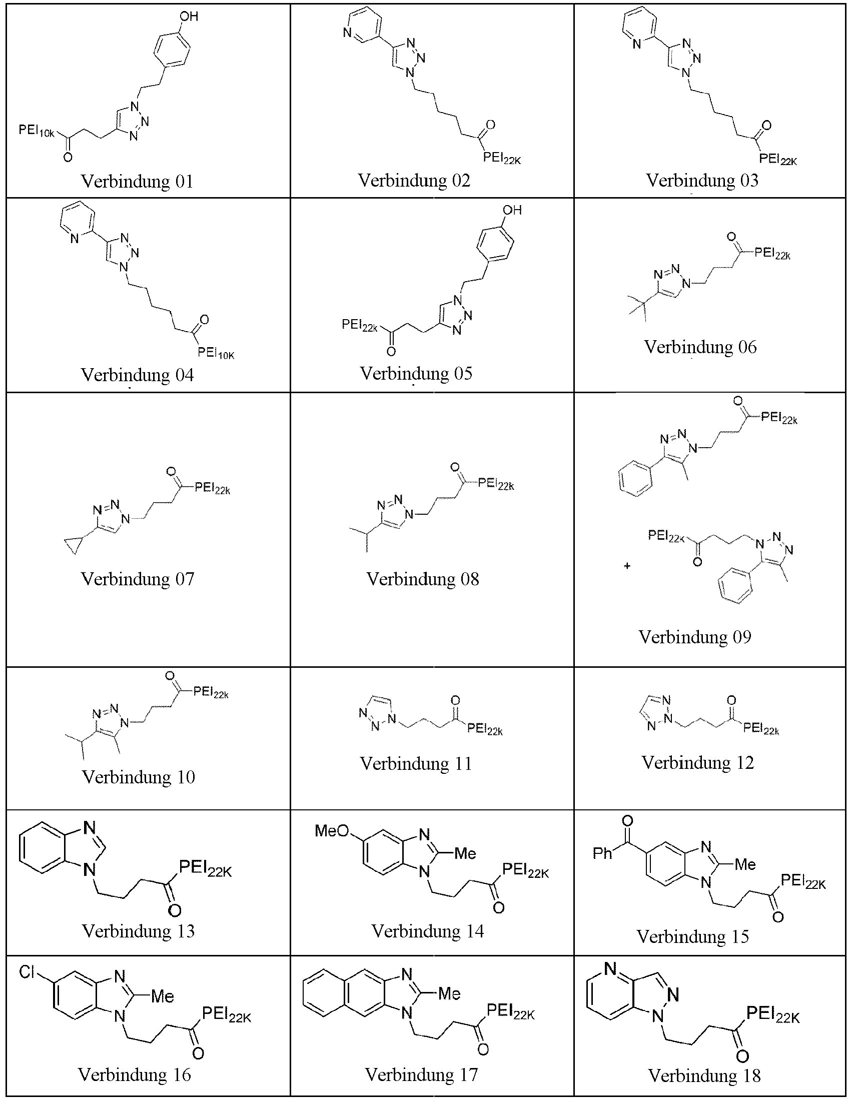

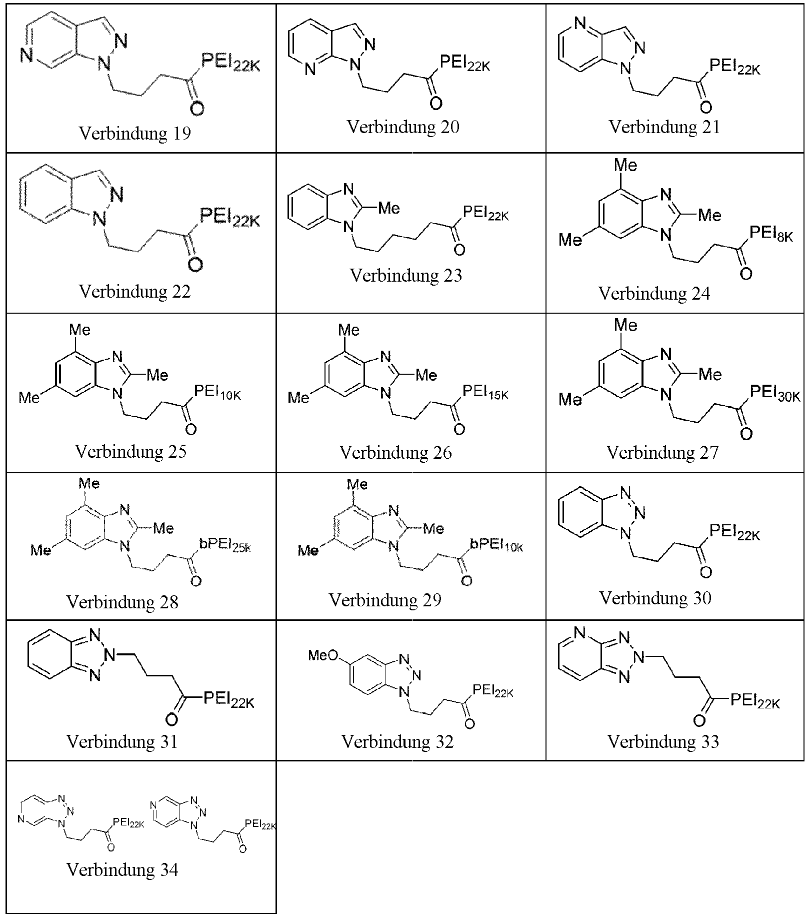

7. Verfahren nach einem der Ansprüche 1 bis 6, in dem die biologische Matrix ausgewählt wird aus der Gruppe bestehend aus einem Zellkulturmedium, insbesondere einem Kulturmedium aus eukaryotischen Zellen, insbesondere Zellen in Suspension oder adhärente Zellen, einem Puffer, einer während des Herstellungs- und des Reinigungsprozesses der rekombinanten Viren verwendeten Lösung, und einer Endzusammensetzung mit den gereinigten rekombinanten Viren, die in einer Endformulierung mit Puffer und pharmazeutisch akzeptablen Hilfsstoffen hergestellt wurden.

8. Verfahren zur Reinigung, zum Nachweis und/oder zur Quantifizierung eines Polyethylenimin (PEI)-basierten Transfektionsreagenzes der allgemeinen Formel (I), wie in einem der Ansprüche 1 bis 6 definiert, in dem das PEI-basierte Transfektionsreagenz der allgemeinen Formel (I) in einer flüssigen Mischung enthalten ist, die eine biologische Matrix umfasst,
wobei die biologische Matrix ein rekombinantes Virus oder Viruspartikel umfasst, die unter Verwendung des PEI-basierten Transfektionsreagenzes der allgemeinen Formel (I) hergestellt wurden,
wobei das Verfahren die folgenden Schritte umfasst:
(a) die Durchführung einer sauren Hydrolyse der flüssigen Mischung gemäß den Ansprüchen 1 bis 7 des Verfahrens,
(b) die Reinigung des in Schritt (a) erhaltenen Reaktionsgemisches, um ein gereinigtes PEI-basiertes Transfektionsreagenz der allgemeinen Formel (I) zu erhalten,
(c) den Nachweis und/oder die Quantifizierung des in Schritt (b) erhaltenen gereinigten PEI-basierten Transfektionsreagenzes der allgemeinen Formel (I).

9. Verfahren nach Anspruch 8, in dem Schritt (c) unter Verwendung einer analytischen Technik der Hochleistungsflüssigkeitschromatographie (HPLC) oder der Ultrahochleistungsflüssigkeitschromatographie (UHPLC), vorzugsweise der UHPLC, durchgeführt wird.

10. Verfahren nach Anspruch 8 oder 9, in dem das PEI-basierte Transfektionsreagenz der allgemeinen Formel (I) aus Schritt (c) mit einer Nachweisgrenze (LOD) zwischen 1 ppm und 1000 ppm und/oder einer Quantifizierungsgrenze (LOQ) zwischen 1 ppm und 1000 ppm nachgewiesen wird.

11. Verfahren nach einem der Ansprüche 1 bis 10, in dem das PEI-basierte Transfektionsreagenz der allgemeinen Formel (I) während des Herstellungsprozesses der rekombinanten Viren in einer biologischen Matrix nachweisbar ist, wobei die biologische Matrix ausgewählt wird aus der Gruppe bestehend aus einem Zellkulturmedium, insbesondere einem Kulturmedium aus eukaryotischen Zellen, insbesondere Zellen in Suspension oder adhärenten Zellen, einem Puffer, einer während des Herstellungs- und des Reinigungsprozesses der rekombinanten Viren verwendeten Lösung, und einer Endzusammensetzung mit den gereinigten Viren, die in einer Endformulierung mit Puffer und pharmazeutisch akzeptablen Hilfsstoffen hergestellt wurden.

12. Verfahren nach einem der Ansprüche 1 bis 11, in dem das rekombinante Virus ausgewählt wird aus der Gruppe bestehend aus einem Adeno-assoziierten Virus (AAV), einem Lentivirus (LV), einem Adenovirus, einem onkolytischen Virus und einem Baculovirus, vorzugsweise einem Adeno-assoziiertes Virus (AAV) oder einem Lentivirus (LV), noch bevorzugter einem Adeno-assoziiertes Virus (AAV).

13. Verfahren nach einem der Ansprüche 6 bis 12, in dem das PEI-basierte Transfektionsreagenz der allgemeinen Formel (I) eine Verbindung ist, die ausgewählt wird aus der Gruppe der Verbindungen 01, 02, 03, 04, 05, 06, 07, 08, 09, 10, 11, 12, 30, 31, 32, 33 und 34, und das rekombinante Virus ein Adeno-assoziiertes Virus (AAV) ist.

## Revendications

1. Procédé d'hydrolyse acide d'un mélange liquide comprenant une matrice biologique et un réactif de transfection à base de polyéthylèneimine (PEI),
dans lequel la matrice biologique comprend un virus recombinant ou des particules de type virus produites en utilisant le réactif de transfection à base de PEI,
dans lequel le procédé comprend l'étape d'incubation du mélange liquide comprenant la matrice biologique dans une solution aqueuse comprenant de 0,1 % à 10 % (v/v) d'acide chlorhydrique (HCl) à une température comprise entre 60 °C et 110 °C pendant une période comprise entre 2 heures et 24 heures, de préférence une solution aqueuse comprenant 0,1 % (v/v) de HCl à une température de 110 °C pendant 2 heures ou une solution aqueuse comprenant 1 % (v/v) de HCl à une température comprise entre 60 °C et 80 °C pendant 2 heures,
dans lequel ladite hydrolyse acide ne dégrade pas le réactif de transfection à base de PEI, et
dans lequel le réactif de transfection à base de PEI est de formule générale (I) ou un de ses sels acceptables : 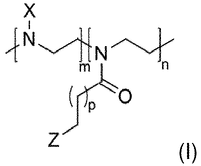
dans laquelle :
- m représente un nombre entier compris entre 27 et 1200 et n représente un nombre entier compris entre 3 et 600, à condition que n soit inférieur à m et que la somme de m+n soit comprise entre 30 et 1200,
- X représente H ou un groupe de formule : 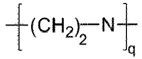
dans laquelle q représente un nombre entier compris entre 10 et 800,
- p représente un nombre compris entre 1 et 4,
- Z représente un groupe de formule : 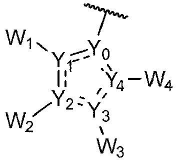
- Y₀, Y₁, Y₂, Y₃ et Y₄, qui peuvent être identiques ou différents, représentent C ou N, à condition qu'au moins deux, mais pas plus de trois, de Y₀, Y₁, Y₂, Y₃ et Y₄ soient N,
- W₁, W₂, W₃ et W₄, qui peuvent être identiques ou différents, représentent H, un alkyle en C₁-C₁₈ linéaire ou ramifié, saturé ou insaturé, un aryle en C₆-C₁₈, un aryle en C₆-C₁₈-alkyle en C₁-C₁₈ linéaire ou ramifié, saturé ou insaturé, un hétéroaryle en C₅-C₁₀, un hétéroalkyle en C₂-C₁₈ linéaire ou ramifié, saturé ou insaturé, une amine, une alkylamine en C₁-C₁₈ linéaire ou ramifiée, saturée ou insaturée, un alcoxy en C₁₋C₁₂ ; ou (i) W₁ et W₂ ou (ii) W₂ et W₃ ou (iii) W₃ et W₄ forment ensemble un aryle à six chaînons fusionné, éventuellement substitué, ou un hétéroaryle à six chaînons fusionné, éventuellement substitué, ne contenant pas plus d'un atome N, à condition qu'au moins un, mais pas plus de deux, de W₁, W₂, W₃ et W₄ soit, ou soient, absents.

2. Procédé selon la revendication 1, dans lequel W₁, W₂, W₃ et W₄, qui peuvent être identiques ou différents, représentent H, méthyle, cyclopropyle, isopropyle, tert-butyle, phényle, benzyle, 2-pyridine, 3-pyridine, ou 4-hydroxyphényl ; ou dans lequel (i) W₁ et W₂ ou (ii) W₂ et W₃ ou (iii) W₃ et W₄ forment ensemble un phényle fusionné ; un phényle fusionné substitué par un groupe méthyle, en particulier deux groupes méthyle, un groupe méthoxy, un carboxyphényl ou Cl ; un naphtalène fusionné ; une 2-pyridine fusionnée ; ou une 3-pyridine fusionnée.

3. Procédé selon la revendication 1 ou 2, dans lequel X représente H.

4. Procédé selon l'une quelconque des revendications 1 à 3, dans lequel le réactif de transfection à base de PEI de formule générale (I) a un taux de greffage défini comme (n/(m+n))*100, et dans lequel le taux de greffage est compris entre 1 et 50 %, de préférence entre 5 et 30 %, et plus préférentiellement de 20 %.

5. Procédé selon l'une quelconque des revendications 1 à 4, dans lequel le squelette polymère PEI a une masse moléculaire moyenne (Mw) comprise entre 1 kDa et 50 kDa, de préférence comprise entre 5 kDa et 30 kDa ou entre 10 kDa et 25 kDa, plus préférentiellement le squelette polymère PEI a une masse moléculaire moyenne (Mw) de 8, 10, 15, 22, 25 ou 30 kDa, de préférence de 22 kDa.

6. Procédé selon l'une quelconque des revendications 1 à 5, dans lequel le réactif de transfection à base de PEI de formule générale (I) est choisi dans le groupe consistant en les composés suivants :

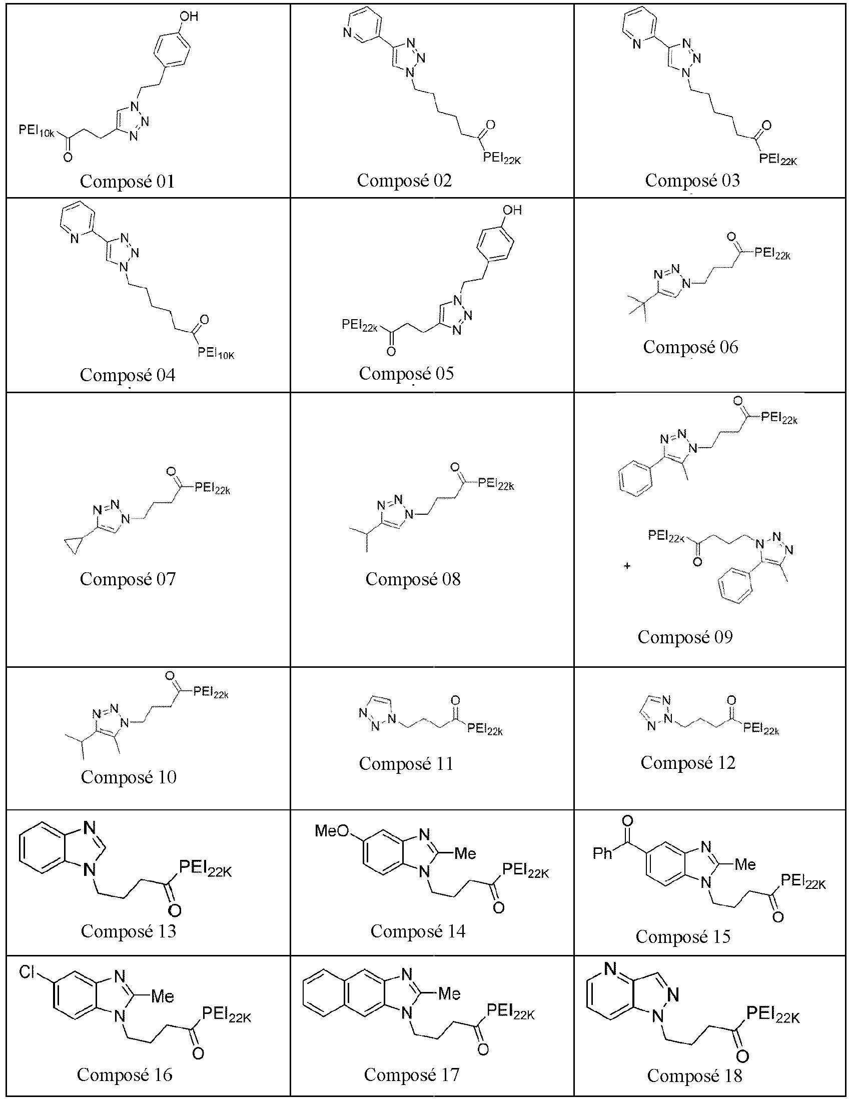

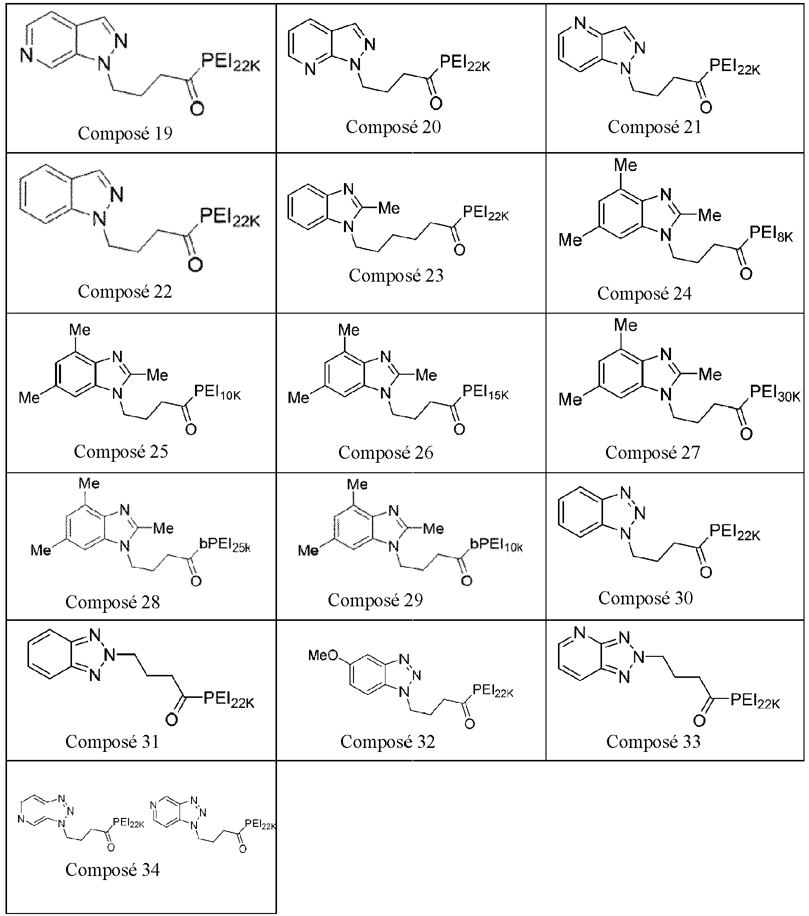

7. Procédé selon l'une quelconque des revendications 1 à 6, dans lequel la matrice biologique est choisie dans le groupe consistant en un milieu de culture cellulaire, en particulier un milieu de culture de cellules eucaryotes, en particulier des cellules en suspension ou des cellules adhérentes, un tampon, une solution utilisée pendant le processus de fabrication et le processus de purification des virus recombinants, et une composition finale comprenant les virus recombinants purifiés fabriqués dans une formulation finale comprenant un tampon et des excipients acceptables sur le plan pharmaceutique.

8. Procédé de purification, de détection et/ou de quantification d'un réactif de transfection à base de polyéthylèneimine (PEI) de formule générale (I) tel que défini dans l'une quelconque des revendications 1 à 6, dans lequel le réactif de transfection à base de PEI de formule générale (I) est compris dans un mélange liquide comprenant une matrice biologique,
dans lequel la matrice biologique comprend un virus recombinant ou des particules de type virus produites en utilisant le réactif de transfection à base de PEI de formule générale (I),
dans lequel le procédé comprend les étapes de :
(a) réalisation d'une hydrolyse acide du mélange liquide selon le procédé selon l'une quelconque des revendications 1 à 7,
(b) purification du mélange réactionnel obtenu à l'étape (a) afin d'obtenir un réactif de transfection à base de PEI purifié de formule générale (I),
(c) détection et/ou quantification du réactif de transfection à base de PEI purifié de formule générale (I) obtenu à l'étape (b).

9. Procédé selon la revendication 8, dans lequel l'étape (c) est réalisée en utilisant une technique analytique de chromatographie liquide à haute performance (HPLC) ou de chromatographie liquide à ultra-haute performance (UHPLC), de préférence l'UHPLC.

10. Procédé selon la revendication 8 ou 9, dans lequel le réactif de transfection à base de PEI de formule générale (I) de l'étape (c) est détecté avec une limite de détection (LOD) comprise entre 1 ppm et 1000 ppm, et/ou une limite de quantification (LOQ) comprise entre 1 ppm et 1000 ppm.

11. Procédé selon l'une quelconque des revendications 1 à 10, dans lequel le réactif de transfection à base de PEI de formule générale (I) est détectable dans une matrice biologique au cours du processus de fabrication des virus recombinants, dans lequel la matrice biologique est choisie dans le groupe consistant en un milieu de culture cellulaire, en particulier un milieu de culture de cellules eucaryotes, en particulier des cellules en suspension ou des cellules adhérentes, un tampon, une solution utilisée pendant le processus de fabrication et le processus de purification des virus recombinants, et une composition finale comprenant des virus purifiés dans une formulation finale comprenant un tampon et des excipients acceptables sur le plan pharmaceutique.

12. Procédé selon l'une quelconque des revendications 1 à 11, dans lequel le virus recombinant est choisi dans le groupe consistant en un virus adéno-associé (AAV), un lentivirus (LV), un adénovirus, un virus oncolytique et un baculovirus, de préférence un virus adéno-associé (AAV) ou un lentivirus (LV), plus préférentiellement un virus adéno-associé (AAV).

13. Procédé selon l'une quelconque des revendications 6 à 12, dans lequel le réactif de transfection à base de PEI de formule générale (I) est un composé choisi dans le groupe consistant en les composés 01, 02, 03, 04, 05, 06, 07, 08, 09, 10, 11, 12, 30, 31, 32, 33 et 34, et le virus recombinant est un virus adéno-associé (AAV).
